(19) Europäisches Patentamt
European Patent Office
Office européen des brevets

(11) **EP 2 917 326 B1**

(12) **EUROPEAN PATENT SPECIFICATION**

(45) Date of publication and mention
of the grant of the patent:
**06.03.2019 Bulletin 2019/10**

(21) Application number: **13792291.0**

(22) Date of filing: **07.11.2013**

(51) Int Cl.:
***C12M 1/32*** *(2006.01)*     ***C12M 1/00*** *(2006.01)*
***C12M 1/34*** *(2006.01)*

(86) International application number:
**PCT/EP2013/073307**

(87) International publication number:
**WO 2014/072432 (15.05.2014 Gazette 2014/20)**

(54) **CELL CULTURE DEVICE FOR GENERATING AND CULTIVATING CELL AGGREGATES, METHOD OF PRODUCING SAID DEVICE AND USE OF SAID DEVICE**

ZELLKULTURVORRICHTUNG ZUR ERZEUGUNG UND KULTIVIERUNG VON ZELLAGGREGATEN, VERFAHREN ZUR HERSTELLUNG DIESER VORRICHTUNG UND VERWENDUNG DIESER VORRICHTUNG

DISPOSITIF DE CULTURE CELLULAIRE POUR LA GÉNÉRATION ET LA CULTURE D'AGRÉGATS CELLULAIRES, PROCÉDÉ DE PRODUCTION DUDIT DISPOSITIF ET UTILISATION DUDIT DISPOSITIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2012 GB 201220065**

(43) Date of publication of application:
**16.09.2015 Bulletin 2015/38**

(73) Proprietor: **anfass Life Technologies AG**
**4500 Solothurn (CH)**

(72) Inventors:
• **THOMSEN, Andreas**
**79341 Kenzingen (DE)**
• **NANKO, Norbert**
**79108 Freiburg (DE)**

• **NIEDERMANN, Gabriele**
**79104 Freiburg (DE)**
• **HENKE, Michael**
**79114 Freiburg (DE)**

(74) Representative: **Frei Patent Attorneys**
**Frei Patentanwaltsbüro AG**
**Postfach**
**8032 Zürich (CH)**

(56) References cited:
**EP-A1- 2 230 014          WO-A1-00/29110**
**WO-A1-91/06624          WO-A1-98/31466**
**WO-A1-2008/106771    WO-A1-2009/154466**
**WO-A2-2007/087402    WO-A2-2010/142755**
**US-A1- 2007 235 902    US-A1- 2009 298 116**

**Description**

**FIELD OF THE INVENTION**

The present invention relates to the field of cell biology and a structured cell culture device, which may be used in said field. In particular, the present invention relates to a device for generating and cultivating cell aggregates, a method for producing a device for generating and cultivating cell aggregates, the use of a device for generating and cultivating cell aggregates and an in-vitro method of determining the influence of a compound or of irradiation on the growth of cells using a device.

**BACKGROUND OF THE INVENTION**

At present, the majority of cell biological studies are carried out with monolayer cell cultures, wherein the monolayer cell cultures are *inter alia* used for studies relating to tumour and non-tumour cells. In such two-dimensional cell cultures, cells grow on a widely spread base, which facilitates experimental conditions and allows for proper microscopic analysis of the cells. However, in such monolayer cell cultures, the behaviour of the cells differs from the behaviour of the cells in the original tissue. For example, the cells are not able to build-up a three-dimensional network such that the exchange of signals between cells and the generation of an extracellular matrix is strongly restricted. Furthermore, due to the direct contact of all cells with the culture medium, an artificial situation is studied in most cases, which does not correspond to the physiological, i.e. the in-vivo situation of the cells.

Different approaches for generating three-dimensional cell cultures have been developed up to now. A first technique is called spinner flask culture, wherein a cell suspension is permanently kept moving such that the cells adhere to each other. Furthermore, several approaches have been developed which make use of micro-patterned surfaces. Therein, photolithographic etching of silicon plates and also of silicon dioxide plates is used. The provided relief corresponds, for example, to a honey-comb architecture.

Several publications provide techniques for generation of multicellular aggregates from cell suspensions. This state of the art analysis is restricted to methods that rely on cell sedimentation into spatially separated compartments.

In the hanging drop technique, this compartment is formed by the air-liquid interface of the drop itself. An early application of this technique was published by Robert Koch, who used it for clonal expansion of bacteria in the 1850s. Decades later, the technique was adapted for eukaryotic cell culture, e.g. to generate embryoid bodies from embryonic stem cells. While the original technique is labour-intensive, the recent development of standardized hanging drop arrays enable large scale, robot-assisted spheroid generation and cultivation[1]. However, many cell types fail to form spheroids in the hanging drop setting, but remain as flattened cell masses that are likely to disintegrate into smaller aggregates upon manipulation. In addition, volume of drops is restricted to less than 50 $\mu$l, which limits cell supply with culture medium and size of spheroids.

Also known in the state of the art is the liquid overlay technique which uses multiwell cell culture plates in which the bottom of each well is coated with a cell-repellent layer that prevents cell attachment. Either the bottom of the wells (V-bottom, U-bottom) or the top surface of the cell-repellent hydrogel provides a concave surface that funnels sedimenting cells to the lowest point of the well, where cells may self-assemble into three-dimensional multicellular aggregates, which can be kept in culture for several weeks and may grow to spheroids of up to 1 mm in diameter, depending from cell type and culture conditions[2]. As a limitation, similar as in the hanging drop technique, cell types having weak cell-to-cell contacts, e.g. most hematopoietic cells and many tumour cell lines, usually do not form spheroids with this technique. However, spheroid formation may be enhanced by addition of extracellular matrix extracts[3], but such supplement could influence treatment response and therefore might be unwanted in certain experimental settings.

Another method to assemble cells into spheroids has been published by Fukuda et al. [5], who used a programmable micromilling system to create a multitude of cylindrical cavities in a polystyrene Petri dish, resulting in a microarray for generation and culture of hepatocyte spheroids. Cavities had a diameter of 100, 300 or 500 $\mu$m and a depth of 50, 100 or 200 $\mu$m. When applied in rotational culture, one of the fabricated cavity sizes (300 $\mu$m diameter, 100 $\mu$m depth) yielded one hepatocyte spheroid each cavity, while other sizes resulted in less even cell distribution. In summary, this microarray showed to be useful for generation and cultivation of hepatocyte spheroids, with the advantageous feature that the favourable cavity size prevented "dislocation" of spheroids even in rotational culture and with non-adhesion treated polystryrene as substrate. However, the micromilling system had to run for 60 minutes to prepare one single Petri dish, which is not acceptable for a single use article.

One possibility to overcome such time-consuming fabrication method would be replica moulding, e.g. using poly(dimethylsiloxane) (PDMS) stamps as described by Tang et al. [4] in 2003. They presented a method to mould and release hydrogels, primarily using the technique to fabricate microstructures from cell-adhesive hydrogels like, but also reported to process agarose gels in the same way.

Napolitano et al. [6] focussed on replica moulding of cell-repellent hydrogels, (mainly agarose and polyacrylamide gels,) producing hydrogel devices with a multitude of recessions to produce multicellular spheroids from cell suspensions, but also more complex cell aggregate geometries as honeycomb structures or toroids. This may be seen as a substantial improvement of the liquid overlay technique, as the method allows the efficient generation of multiple spheroids with one single pipetting step. However, generation of uniform spheroids with this technique is limited, mainly due to the fact that not all cells actually reach a cavity but remain on the surface between cavities.

However, as the previously listed methods, this technique is restricted to cells that form spheroids, so that cells with weak cell-to-cell contacts fail to be cultured as 3D multicellular aggregates with this technique.

WO 91/06624 A1 is known in the state of the art and discloses a tissue dish with culture wells with a design that is supposed to prevent surfaces that tend to trap cells and shall assist to cannel cells into the nutrient medium. The dish does not comprises a hydrogel substrate.

Wo 2008/106771 A1 is also known in the state of the art and discloses a multiwell plate for culturing and preparing cell aggregates. The wells are made of PDMS material. An exact volume determination is not possible due to the specific geometry of the single wells.

WO 2010/142755 A2 is a document known in the state of the art and discloses a device for in vitro aggregation of cells with a special well geometry.

The document WO 00/29110 A1 is also known in the state of the art and discloses a matrix plate for use in sample analysis.

EP 2 230 014 A1 discloses devices and methods for the formation of cell aggregates, especially of pluripotent stem cells. The wells show a special geometry but are not deep enough to enable an ongoing cultivation.

WO 98/31466 A1 discloses a multi-well assay plate which is formed using an injection molding technique. The geometry of the wells does not allow an easy volume determination which is a disadvantage of the disclosed device.

WO 2009/154466 A1 belongs to the state of the art and describes an approach to constructing cellular aggregates in vitro and their use in methods for producing 3D-tissue constructs.

US 2007/235902 A1 belongs to the known state of the art and discloses a microstructured tool having a microstructured layer and a microstructured surface.

However, all of the already known techniques for providing micro-patterned surfaces entail specific technical disadvantages such that no commercial application of a micro-structured cell culture device has gained a broad acceptance in the life science field thus far. Further, a high throughput production method of such devices is not available at present and the read-out of the known platforms is elaborate and to some extent impractical. An exact and easy volume determination is not possible with the devices known in the state of the art.

## SUMMARY OF THE INVENTION

Thus, there is a need to improve current cell cultivation and analysis means. In particular, there is a need for providing structured devices for three-dimensional cell cultivation and analysis with high mechanical precision and different geometries. Further, there is also a need of improved readout possibilities. The present invention matches said needs.

The object of the present invention may be seen in the provision of an advantageous cell culture device, an advantageous production method of said cell culture device, advantageous uses of said cell culture device, and an advantageous in vitro-method of determining the influence of a compound or substance or irradiation on the growth of cells.

The object of the present invention is solved by the subject-matter of the independent claims. Further embodiments and advantages of the invention are incorporated in the dependent claims.

The herein described exemplary embodiments similarly pertain to the cell culture device for generating and cultivating cell aggregates, the method of producing said device, the use of said device, and the in vitro-method as disclosed herein.

Synergistic effects may arise from different combinations of different embodiments, although they might not be described in detail hereinafter.

Furthermore, it shall be noted that all embodiments of the present invention concerning a method might be carried out with the order of the steps as described; nevertheless, this has not to be the only and essential order of the steps of the method of the present invention. The skilled person gathers from the following disclosure that different orders and combinations of the method steps described herein can be combined, unless explicitly mentioned to the contrary.

In a first preferred embodiment of the invention a Cell culture device for generating and cultivating cell aggregates, the device comprising,

- a cell repellent hydrogel substrate,
- a surface,
- at least a first and a second recession for receiving cells for generating and cultivating cell aggregates,

wherein the first and the second recessions are spatially separated, and

wherein the surface is a sloped surface and

wherein the first and the second recessions comprise an upper part and a bottom part and wherein the upper part has the shape of a truncated cone with an opening angle of 15 to 30° and the ratio of the depth of the upper part to the diameter of the widest end of the upper part is at least 1.5 : 1.

It is in particular preferred that the surface between the first and the second recession is a sloped surface.

The widest end of the upper part is the part of the truncated cone which has the greatest distance from the bottom part.

A cell culture device according to the invention can also be called a matrix, a cell culture apparatus or a cell culture vessel.

"Cell aggregate" in the context of the invention means the accumulation of two or more cells. It is preferred that according to this invention this accumulation includes both cell-to-cell adhesion, as it takes place in spheroid formation, and adhesion-independent accumulation of cells. The term "cell aggregates" as used in this respect *inter alia* means especially that the cells are present in a three-dimensional shape and not in the form of a mono-layer (i.e. a two-dimensional shape).

In contrast to spheroid forming cells, e.g. embryonic and mesenchymal stem cells, fibroblasts and some tumour cell lines, many cell types form only weak intercellular adhesions, so that the resulting multicellular aggregates are neither spherical nor mechanically stabile. Due to the special shape of the recessions of the invention it is possible not only to cultivate spheroid forming cells but also to cultivate cell aggregates which are not spherical or mechanically stabile. One of the advantages of the invention is that these cells can be cultivated in a three dimensional manner. Therefore the invention improves the cultivation environment for many different cell types by culturing the cells in a context that closely resembles the tissue of origin.

Due to the shape of the device gravity can be used effectively when distributing a cell suspension over the cell culture device leading to a fast and proper movement of the single cells into at least one of the first and second recession in a uniform or at least substantially uniform manner. In the context of the present invention, said distribution of the cells will also be referred to herein as loading one or more recessions with cells. A major benefit of the invention is that due to the sloped surface it can advantageously be avoided that cells sediment and remain on that surface, so that and all or substantially all cells of the cell suspension are properly distributed into the desired recessions. Therefore it is possible to generate and cultivate uniform or substantially uniform cell aggregates. This is of high importance when it comes to different kinds of analysis or substance testing. Since the cultivation of very uneven cell aggregates is a major problem in the state of the art the device of the invention provides a simple but superior solution to this problem.

The cell culture device of the present invention may therefore be used to generate uniform cell aggregates. The term "uniform" as used in this respect refers to substantially identical (particularly with respect to the size) cell aggregates.

In terms of the invention "surface" describes in particular the part of the device above the upper part of the truncated cone shaped recessions. A sloped surface according to the invention is in particular a surface which does not allow cells to sediment on. This is achieved by the shape of the surface. "Sloped" in terms of the invention in particular refers to curved, convex, declining or bent surfaces. In contrast, a "horizontal surface" describes a surface which is parallel to the bottom of the device, which is not desired in the invention.

The features of the recessions, especially the opening angle of 15 to 30° and a the ratio of the depth of the upper part to the diameter of the widest end of the upper part of at least 1.5 : 1, are especially advantageous. Due to these measurements the invention can present a device which is ideal not only for generating but also for cultivation cell aggregates. Devices described in the state of the art are either not deep enough or have an opening angle which is too wide for a robust 3D cultivation of cells. Therefore it is for example not possible to change the cell culture medium without flushing cells out of the device or transferring them into another recession. This problem is solved by the device of the invention.

Preferred are opening angles of 20 to 25°.

Also preferred is that the ratio of the depth of the upper part to the diameter of the widest end of the upper part is at least 1.5:1, more preferred 2.5:1 most preferred 2:1. It was surprising that a ratio from 2:1 or almost 2:1 leads to a geometry which is superior to the known devices. It is also possible that the ratio is 4:1, 5:1 or at least 10:1. The person skilled in the art is able to choose the best suited ratio for each approach.

The present device may thus be used to obtain substantially identical three-dimensional cell aggregates in the recessions of the device. These are two important aspects in testing methods employing *in vitro* cell cultures. Thus, in order to obtain reliable results from a test method, such as e.g. a screening procedure for compounds inhibiting cell growth, it appears crucial to establish a system, wherein standardized procedures and components are used. For a mono-layer cell culture system, this e.g. refers to an identical number of cells in each well. In the present case, this refers to the cell aggregates being present as uniform or substantially uniform cell aggregates in the recessions. It also seems to be crucial to have the cells present in a situation as close as possible to the physiological situation (e.g. regarding oxygen and nutrient support or proliferation). In this respect, three-dimensional cell aggregates appear superior over e.g. mono-layer cells since such aggregates more closely resemble the situation within bodies, such as the human body (see e.g. "biology's new dimension, NATURE, vol. 424, August 2003, pages 870-872).

As will become clear from the following explanations, the present invention allows for the production of such a microstructured cell culture device for improved cell analysis with proper dimensions and desired precision. Different further aspects of the geometry and shape of the recessions and surface can be added to herein described embodiment. As will become clear from further exemplary embodiments described herein, different aspects of the cell culture device can be combined with the feature that the surface between the two recessions causes a transport of cells by gravity from surface into at least one of the first recession and the second recession. In other words, the surface between the first recession and the second recession is sloped and preferably does not have any horizontal parts or portions, but preferred has only sloped or curved elements. In particular, the cell culture device of the present invention can be used with cells provided as a cell suspension.

Not having any horizontal parts or portions in terms of the invention means in particular that the surface is shaped in a way that cells, especially sedimenting cells in cell culture medium will not remain on that surface because of the sloped and/or curved shape.

Cells, which have been loaded into at least the first recession, are present in the bottom part of said recession in a manner, which forces the generation of cell aggregates. Thus, the cells are present therein in a stacked and tight manner and separated from cells present e.g. in a second recession. This results in the formation of cell-cell contacts among the cells present in said recession and the formation of a three-dimensional cell aggregate in said recession, wherein only the outermost cells are directly exposed to the medium comprising *inter alia* nutrients. Since the cell culture device is made of a hydrogel substrate the device is permeable e.g. for nutrients or other compounds comprised in the medium, there is, however, no limitation with respect to the medium and its ingredients.

The cells cultured in a device of the invention are preferably selected from the group of biological and functional units of multicellular organisms, primary cells, that were recently removed from the healthy or diseased organism, cells that have been subjected to manipulations and selecting steps after original isolation, e.g. expansion of cell number in vitro or in vivo, selection of subpopulations with special properties, treatment with compounds or growth factors, exposure to physical effects, labelling of cells or parts thereof, or application of genetic modifications. The skilled person familiar with cell biology and cell culture methods will gather from these examples that cells may have been subjected to a broad spectrum of treatments.

Another advantage of the invention is that the devices comprises, preferred is made of, a hydrogel substrate which is cell repellent. This improves the formation of uniform cell aggregates because all cells or nearly all cells reach the bottom part of one recession. Single cells do not attach to the side walls or the bottom of the recessions. In combination with the sloped surface this feature supports the uniformity of the generated cell aggregates.

According to a preferred embodiment of the invention, the part between two or more recessions can also be called partition wall. The partition wall has a shape which facilitates a transport of cells by gravity from a distal end of the partition wall into at least one of the first recession and the second recession. The distal end of the partition wall is also part of the sloped surface, preferred without any horizontal parts.

The size and the form of the cell aggregates are of course defined by the geometrical settings of the recession (such as e.g. the shape of the bottom part and the width of the recession). If a cell culture device comprises not only one but several recessions, which are identical in their geometrical settings, uniform cell aggregates can be obtained in said recessions if such a device is loaded with a cell suspension. This is an especially preferred embodiment of the present invention.

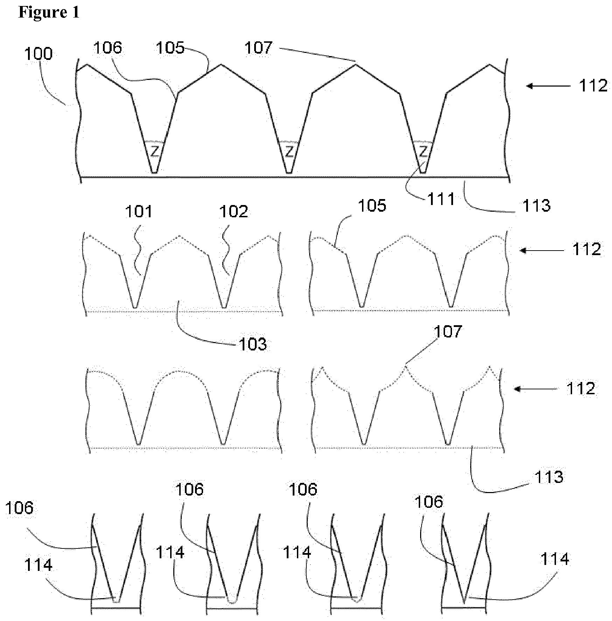

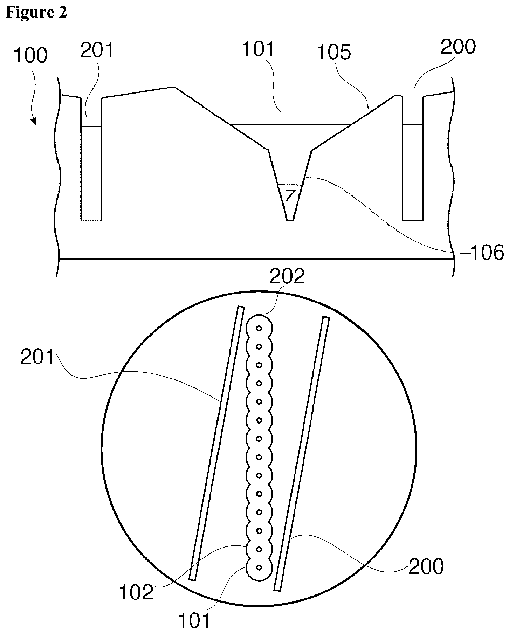

The present invention provides for the advantage that the sedimentation and accumulation of cells between the first and the second recession is avoided. Due to the sloped surface, ideally without any horizontal parts, cells get automatically transported into the desired direction. It is preferred that the surface comprises at least one sloped side wall. In this embodiment the sloped side wall is directed into on recession, therefore the sloped side wall is inwardly directed to one recession. It is especially preferred that the surface between at least two, preferred four recessions is shaped as a tapered tip. Based on the production method as presented herein, such sloped sections and walls of the surface can be produced on the desired scale. For example, this geometry can be provided for a cell culture device in which adjacent or neighboured surface parts between recessions are distanced from each other by, for example, 1 mm as can be seen in Fig. 1, or 2 mm, or more than 2 mm, or below 1 mm. Also other dimensions are possible, e.g. as listed in Table 1. This aspect will be explained in more detail hereinafter. Also, a width of the recession in the range of a single cell, which typically has a diameter in the range of between 0.008 and 0.02 mm, can be provided, as well as larger diameters such as e.g. 0.1 mm, or 0.2 mm or 0.3 mm or 0.5 mm.

It is also preferred that the surface comprises convex areas, which prevents the sedimentation of cells between two recessions.

Generally, the width of a recession depends on the type of cells to be analyzed and the goal of the analysis: if cell aggregates consisting of a few cells only are to be analyzed, the width of a recession should be rather small (this may apply for example to assays, wherein stem cells or progenitor cells are seeded in a clonal density). If larger cell aggregates should be analyzed, a larger width should of course be applied (this may apply for example to assays, wherein chondrocytes are tested; these cells require to be present in large aggregates for proper differentiation and proper production of extracellular matrix). Further, the size of the cells to be analyzed needs to be taken into account in this respect. Suitable widths range from 10 $\mu$m to 1 mm. In this context width refers to the width of the bottom part of a recession.

The distance between the recessions also depends on the type of cells to be analyzed, but further on the size of the cell aggregates: the larger the cell aggregates, the more supply with nutrients etc. comprised in the medium is needed. If the distance between the recessions is small, supply-problems may arise. Thus, in case of large cell aggregates, it may be more appropriate to select a larger distance between the recessions (resulting in broader partition walls between the recessions). If slow-growing cells are used, supply might not be limiting; thus, small distances between the recessions may then be used.

Further, the amount of cells in the cell suspension used for loading the device needs to be considered: the higher the amount of cells, the higher the consumption of nutrients etc. such that a larger width and distance may be more appropriate.

For a cell culture device according to the present invention, the following distances between the recessions and widths of a recession may be used: distances ranging from 0.25 mm (small cell aggregates, slow-growing cells) to 5 mm (larger cell aggregates, fast-growing cells), wherein the width of a recession may be in the range of 10 $\mu$m (very few cells in suspension) to 1 mm.

The following dimensions have been found as being suitable for most of the experimental approaches using standard cell lines: 1 mm or 2 mm distance between the recessions and 0.2 mm width per recession.

The method of producing a cell culture device according to the present invention, which method will be described hereinafter in more detail, allows for the production of the previously described, different widths of the recessions and the distances between the recessions of the cell culture device. Depending on the desired application and/or cells to be analyzed, the step of processing the solid state body to create a device model can be embodied and adapted respectively.

Furthermore, as it becomes clear throughout the whole description of the figures, the recessions comprise an upper part and a bottom part. The bottom part is located close to a base section of the device. Moreover, if features, functions and configurations of the device, in particular of the first and/or the second recession, the partition wall and/or the surface (especially the surface between at least two recessions), are described herein, this pertains to a device with a plurality of recessions and a plurality of partition walls as well. The skilled person gathers and contemplates from this that the features relating to one element like a first recession or a partition wall or a surface between at least two recessions are described and disclosed herein also for a plurality of recessions and a plurality of partition walls having the same function, feature and configuration. Thus, a device with a plurality of similar or identical recessions and partition walls can be provided by the present invention. Plurality of recession means preferred at least three recessions.

According to another exemplary embodiment of the invention, the surface comprises at least one sloped side wall. In the context of the present invention, the term "slope" shall be understood synonymously to steepness or gradient. This inwardly sloped sidewall of the surface is an embodiment which creates the effect that cells are transported by gravity into at least one of the first recession and the second recession. If desired, a surface between two or more recessions comprises adjacently positioned inwardly sloped sidewalls, such that a tip at the distal end of the surface is provided. Said feature can be seen in Figure 1. As can be seen from Figure 1, the distance between two tips can be 1 millimetre (mm). However, also larger distances or also smaller distances are possible (see above).

It is especially preferred that the truncated cone of the upper part of at least one recession is steeper than the sloped side wall. This feature additionally supports the flow of the cells into the bottom part of the recessions.

Also this feature can e.g. be seen in Figure 1, where the sloped sidewall has a slope, steepness or gradient which is lower than the slope, steepness or gradient of the upper part of the recession.

In other words, the recessions are conically shaped such that the cells, which are inserted in the respective recession, accumulate at the lowest end of the recession. As will become clear from the detailed description hereinafter regarding the production method, such a shape of the recession can be provided in a gel based device, which is highly interesting for cell studies. The dimensions, on which such geometry can be provided, achieve specific advantages for cell analysis.

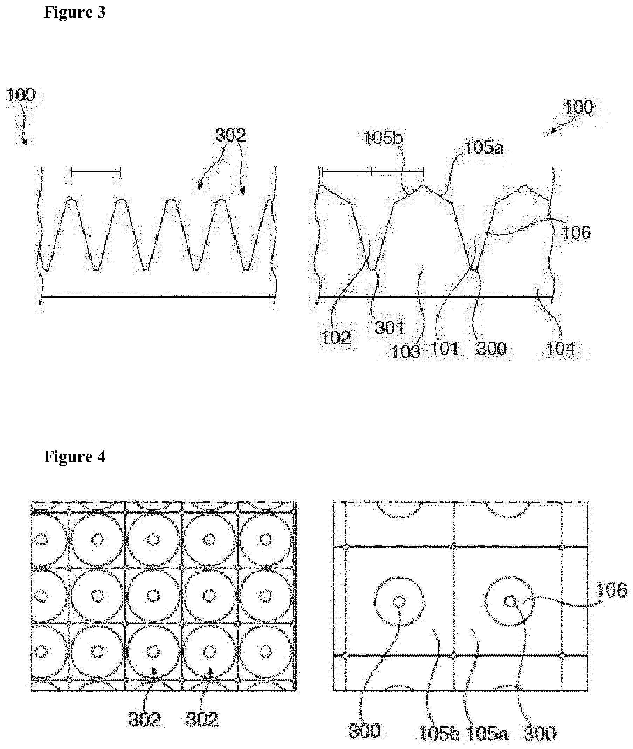

The conical shape is provided such that the recession is tapered towards the base and thus towards the proximal end of the recession. Furthermore, it can be gathered that the bottom of the first and the second recession is flat. In other words, the first and the second recession (and not only the upper part of the recessions) have a shape of a truncated cone. In another exemplary embodiment of the invention, the truncated cone has an exemplary opening angle of 25 degrees (see also Example 1). Generally, the opening angle can be in a range of between 15 and 30 degrees. As can inter alia be derived from Fig. 3, the side walls of a recession in the form of a truncated cone have a defined slope. Generally, it is preferred that this slope is steeper than the sloped sidewalls of the surface. The shape of a truncated cone is advantageous if a simple optical readout is desired in order to determine the volume of the individual cell aggregates. This will be explained in detail below and in the Examples of the present application.

The shape of a truncated cone is also preferred because it is possible to cultivate cells that do not form any spheroids. For these cells the recessions can be used as a measuring cup. Due to the defined shape it is possible to analyse the volume of such cell aggregates in an advantageously simple way. It was very surprising that an opening angle between 15 and 30° is especially suitable for the use as a cell aggregate volumetry unit, similar to the use of a measuring cup.

According to another exemplary embodiment of the invention, the cell culture device is a microstructured device and is preferably made of agarose, alginate or chemical modifications or mixture thereof.

Agarose is an appropriate hydrogel for generating the herein provided devices. Advantages of agarose are, for example, material properties like biocompatibility, optical transparency and being cell repellent. This will be described in even more detail with respect to the description of the production method and the description of the figures. Furthermore, agarose is permeable for components of the medium such that e.g. no supply-problems arise in that respect that the agarose would be a barrier to nutrients or the like.

According to another exemplary embodiment, the cell culture device is made of alginate which is also a hydrogel. Also other hydrogel materials may be used for producing the device, which have desired material properties like being biocompatible, allowing diffusion, optically transparent and cell repellent. In case the biocompatible hydrogel alginate is used, the following may be taken into account: by means of a silicone mould, the device can be cast or moulded. Therein, a Na-alginate solution is poured into the form and a plane gel is provided on top of the form, which gel comprises a calcium chloride solution. Thus, a polymerisation is caused. Curing time may take a few hours, and may take longer than the cooling process of heated agarose. The resulting Ca-alginate gel has the interesting property that it does not melt upon heating; thus it can advantageously be sterilized by autoclaving.

According to another exemplary embodiment of the invention, the device comprises or consists of a mixture of alginate and agarose. This exemplary embodiment of the invention allows for tuning the mechanical properties and the permeability of the gel to the desired extent.

Preferably, the device according to the present invention fails to comprise polyacrylamide and is not formed from polyacrylamide, respectively, due to the toxic characteristics of acrylamide.

The term "microstructured" as used in the present application refers to a structure comprising a three-dimensional architecture with a plurality of geometrically defined and ordered recessions in a micrometer scale.

According to another exemplary embodiment of the invention, the surface between at least two, preferred four, recessions comprises a tapered part ending in a tip. In other words, the partition wall between two or more recessions has a distal tapered section which ends in the tip. This exemplary embodiment may be gathered, e.g., from Figs. 1 and 3.

According to another preferred embodiment of the invention, the cell culture device further comprises a plurality of recessions shaped like the first or the second recession and at least one enlarged recession element for receiving a substance and/or cells, wherein the recessions are located in a way that at least two recessions have a different distance to the enlarged recession element.

In terms of the invention an enlarged recession element is a recession which has a greater volume than the first or the second recession. The enlarged recession element can have any shape and is not limited to the shape of the first or the second recession.

It is also preferred that the enlarged recession element is an elongated recession element for receiving a substance or for receiving cells. The first recession and the second recession are located in the device along a first line. Further, the elongated recession element expands in the device along a second line wherein the first line and the second line are not parallel to each other. The elongated recession element may be embodied as several different sub-elements which are spatially separated. However, it can be also embodied as a continuous object, e.g. as channel, as it is shown in Fig. 2 with reference signs 200 and 201. Therein, it should be noted that the first line and the second line are virtual lines for the sake of describing the location and geometry of the device. Such term "line" shall not be construed in a way that a device necessarily provides for physical line. If desired, the angle may be between 6 degrees and 18 degrees. Also other values may be beneficially applied. Due to the diagonal or inclined elongation of the recession element compared to the first and the second recessions, a gradient in diffusion can be realized. Consequently, a concentration dependent reaction of cell aggregates can be surveyed and studied with this specific device. This aspect of the present invention will be further explained with respect to Fig. 2.

This setup can be used to evaluate if the substance releases one or more agents that affect cell aggregate volumes by diffusion through the hydrogel.

"Substance" in the meaning of this invention comprise in particular a chemical compound (a pure chemical substance), a mixture of several compounds, a biological matter or specimen, which may consist of or may be derived from microbes, fungi, plants, animals or humans, including body fluids and tissues, a liquid, solid or semisolid matter which is to be tested on 3D cell cultures. The latter may be of industrial production or environmental origin, including, but not limited to: drug formulations, cosmetics, food, samples or particles extracted from air, water and soils.

It is also preferred that a plurality of recessions are arranged as one or more rows or concentric circles of recessions, wherein the first recession and the second recession are part of the one or more rows or circles of recessions.

According to another exemplary embodiment of the invention, it is especially preferred that the elongated recession element expands diagonal to the row of conical recessions such that different distances are generated between the elongated recession element and the conical recessions.

It is also preferred that the elongated recession is formed in a different shape, e.g. as a curved channel. The important feature is that there is a gradient in diffusion, which means that some recessions are closer to the elongated recession than others.

According to another exemplary embodiment, the device further comprises at least one breakthrough.

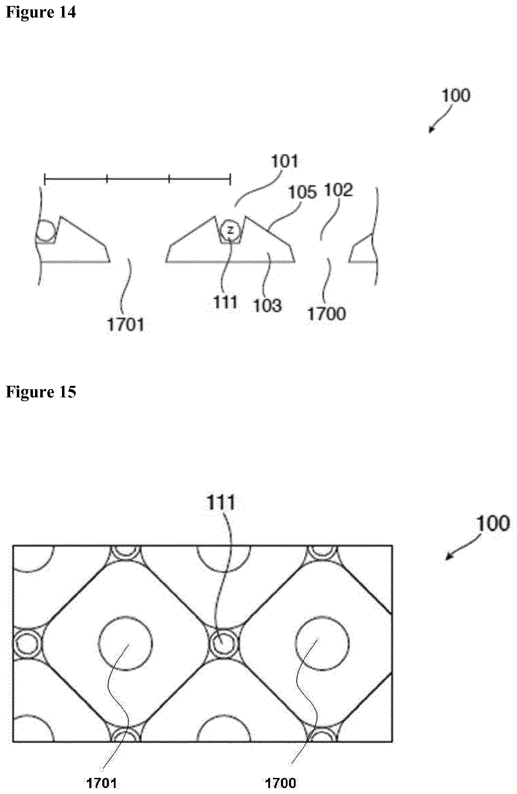

Advantages of this embodiment will be described in detail with respect to Figs. 14 and 15. By means of this device, an analysis and a quantification of cellular invasion within a second, degradable hydrogel (e.g. fibrin or matrigel) can be analysed. It should be noted that the device shown in Fig. 14 can be additionally provided with a chamfer at the end of the cell recession such that a slope for gravity transport into the recession for collecting the cells in element 101 can be provided.

According to another exemplary embodiment of the invention, the breakthrough is configured such that cells, which are not guided into or comprised one of the recessions, are transported by gravity through the breakthrough and thus through the device. The beneficial analysis that can be performed with such a microstructured device will be explained in more detail with respect to Figs. 14 and 15.

According to another exemplary embodiment of the invention, the device has a size, which is larger than 80 cm$^2$. Such a size may be of special practical value. However, it is also possible by means of the present invention to provide larger or smaller devices. If desired, device s with sizes suitable for wells of e.g. 6, 8 or 12 well plates can be provided, such as e.g. with a size of 9.5 cm$^2$ or 3.8 cm$^2$.

According to another exemplary embodiment of the invention, the sloped side wall of the surface has an angle which is selected from the group of angle ranges comprising 20 to 70 degrees, 30 to 60 degrees, 35 to 55 degrees, and 40 to 50 degrees. An angle of 40 degrees may be particularly preferred. Moreover, said angle of the sloped side wall is defined relative to the horizontal direction. As can also be gathered from Fig. 1, the previously defined value of the angle has to be doubled and subtracted from 180 degrees in order to obtain the corresponding opening angle. The opening angle is used herein as the angle, which is defined by the chamfer at the distal end of the recession element shown e.g. in Fig. 1.

According to another exemplary embodiment of the invention, the device has an L-form cross-section. This embodiment has in particular the advantage that the device can be provided in a first position, in which a loading of the first recession is facilitated. If needed, e.g. after polymerization of a second hydrogel has been completed, the L-formed device can be rotated and can be provided such that the first recession is provided in a horizontal position. This may facilitate optical readout means of a microscope or a scanner.

According to another exemplary embodiment of the invention, the device is configured for facilitating the loading of the first recession with cells in a first vertical position of the first recession and is configured for facilitating an optical readout of the first recession in a second horizontal position.

According to another aspect of the present invention, a method of producing a cell culture device for generating and cultivating cell aggregates is provided. The method comprises the steps of providing for a solid state body, processing the solid state body thereby creating a device model, generating a mould by moulding the device model, and casting the device by filling the mould with a solution.

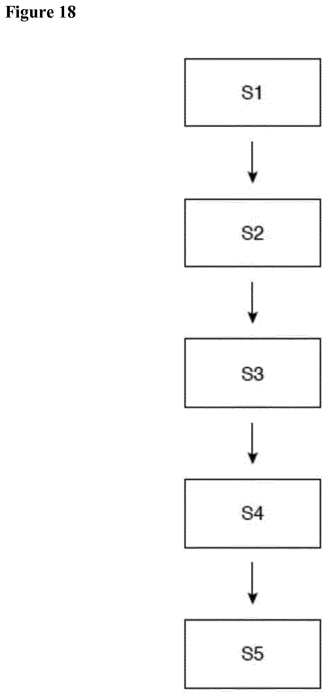

It should be noted that the term "processing" comprises e.g. machining, such as e.g. drilling or milling, electrical discharge machining, electro chemical machining and also non-cutting processes, such as e.g. pressing, punching, embossing or 3D printing. The method steps are described in further detail in connection with Figs. 18 to 23.

In particular, the solid state body may be of a material, which allows the subsequent processing step. For example, a polymer as PMMA, or a metal, as steel, copper, a copper alloy, lead and/or silicon, can be used as material of the solid state body. However, also other materials can be used in connection with the present invention. By means of the presented way of generating the model of the cell culture device, a low cost solution is provided, which allows for the desired cell culture device geometry on the scale and with the dimensions as described herein. In contrast to known processes like photolithography or rapid prototyping the presented production method achieves economical advantages. In particular, specific, very small structures in the cell culture device, like very precise slopes at the partition walls, can be manufactured by means of the presented method. The mould, which is generated by moulding the device model, is the substantially exact negative of the device model, and thus the substantially exact negative of the cell culture device, which is to be produced. As the mould is generated by moulding or forming the device model based on the processed solid state body or from the processed solid state body, a nearly unlimited number of moulds can be generated from the device model. This is a further advantage of the presented method, as a high throughput and commercially relevant production is facilitated, which provides for a very high mechanical precision of the microstructured devices.

It should be noted that the term "moulding" can be embodied in various ways in the meaning of the present invention. This will become more apparent from the following exemplary embodiments. Based on the moulded or formed device model, the product, i.e. the device to be produced, can be casted by filling the desired material into the mould.

According to another exemplary embodiment of the invention, the device mould is generated by curing an addition curing silicone rubber. Addition curing silicone rubbers provide for the advantage to achieve a fast and proper cross-linking such that an appropriate release of the device model from the mould is facilitated.

According to another exemplary embodiment of the invention, the device mould is generated by using condensation-cured silicones.

Depending on the device geometry and the way how the device is released, also elastics, like for example polyurethane, and even completely rigid polymers might be used. Due to the conical geometry of the device and due to the absence of indentations, the device can be released without harming the proper form of the device.

According to another exemplary embodiment of the invention, the silicone rubber is heat-resistant.

According to another exemplary embodiment of the invention, the method further comprises the step of applying vacuum or a centrifugal force by centrifugation during the step of generating the mould by moulding the device model. In order to get rid of air bubbles, the two above mentioned alternatives may be useful. Even very tiny air bubbles are removed by applying centrifugation. The same parameters for centrifugation may be used during centrifugation of the mould with the solution, as will be described hereinafter.

According to another exemplary embodiment of the invention, the method further comprises the step of preheating the mould to a temperature T1 before filling the mould with the solution.

On this preheating step, it is desirable to use heat-resistant silicone rubber. According to another exemplary embodiment of the invention, the temperature T1 may be adjusted to the type of hydrogel used and may be chosen depending on the melting point of the hydrogel. According to another exemplary embodiment of the invention, the temperature T1 (especially for agarose) is selected from the group of temperature ranges comprising 60-95°C, 60-80°C, 62-78°C, 64-76°C, 66-74°C, 68-72°C, 70-80°C, 72-80°C, 74-80°C, 76-80°C, and 60-70°C.

According to another exemplary embodiment of the invention, the method further comprises the step of prehearing the solution to a temperature T2 before filling the mould with the solution. According to another exemplary embodiment of the invention, the temperature T2 is selected from the group of temperature ranges comprising 35-100°C, 40-90°C, 50-80°C, 60-80°C, 72-88°C, 74-86°C, 76-84°C, 78-82°C, 80-90°C, 82-90°C, 84-90°C, 86-90°C, and 88-90°C.

According to another exemplary embodiment of the invention, the solution is an agarose solution. Also, a combination of agarose with other materials as described above is possible in the production method presented herein.

According to another exemplary embodiment of the invention, the agarose solution has a concentration of agarose, which is chosen from the group comprising 1-10%, 2-8%, 3-7%, 2-5%, 2-3% and 2,4% (w/v).

According to another exemplary embodiment of the invention, the step of centrifuging the mould with the solution is provided. Therein, an air bubble reduction is achieved, wherein the same parameters of centrifugation can be used, if desired, as described with respect to the centrifugation during the step of generating the mould by moulding the device model.

According to another exemplary embodiment of the invention, the method comprises the step of centrifuging the mould with a solution. Different parameters for the centrifugation may be chosen. The step of centrifuging the mould may be carried out in a period of one minute, approximately one minute, between a second and one minute, and one second and two minutes. Different accelerations may be applied during centrifugation, e.g. a relative centrifugal force of 2000 g. However, also other values are in the scope of the present invention.

According to another exemplary embodiment of the invention, the method further comprises the step of levelling the solution in the mould.

For example, a glass plate or a similar device may be used in order to reach a levelling of the solution, which is comprised in the mould. Therefore, in an exemplary embodiment of the invention, the levelling is carried out by placing an object onto the mould.

According to another exemplary embodiment of the invention, the method further comprises the step of cooling the mould with the solution after the step of filling. The mould comprising the solution may, for example, be placed on ice or may be inserted into a refrigerator. However, also other cooling techniques may be applied. Depending on the volume and temperature of the solution (e.g. the agarose solution), the release of the solidified or hardened agarose may be possible after e.g. 4 to 10 minutes. Depending on the solution used and the applied cooling temperatures, different cooling time periods can be achieved.

According to another exemplary embodiment of the invention, the method comprises the step of machining the first recession and the second recession into the solid state body.

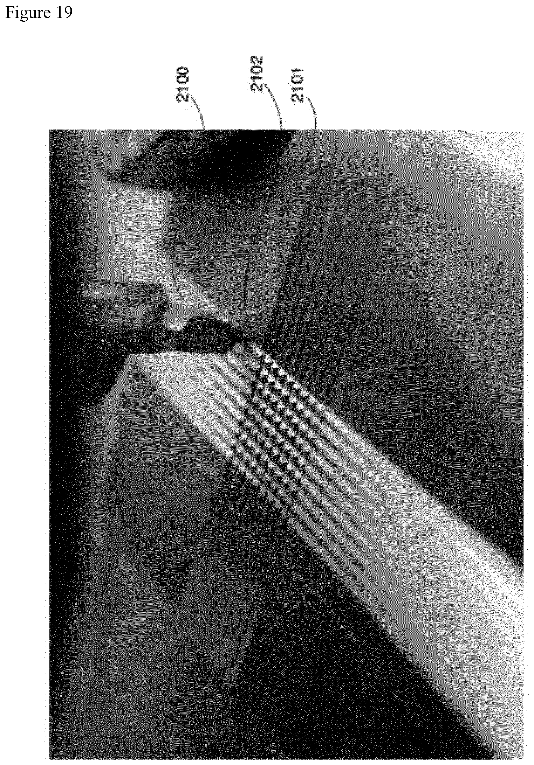

According to another exemplary embodiment of the invention, the step of machining the first and the second recession is carried out by a non-cutting method like pressing or punching, wherein a conical, wedge-shaped (this shape may be particularly preferred for a device with an L-form cross section) or cylindrical punch is pressed or punched into a solid state body. This body may be of a ductile material. It may be the first solid state body but may also be a second solid state body, which was shaped from the first solid state body. This can be gathered from Figs. 19 to 22. For example, lead may be used as such a second solid state body of ductile material, but also other material may be used. In particular, for recessions with small dimensions, like a small height, a small width, and/or small sloped sections, and/or low dimension chamfers, this process and embodiment of the presented method is of particular interest.

According to another exemplary embodiment of the invention, the first recession comprises a first opening and a second recession comprises a second opening. The method of this exemplary embodiment further comprises the step of chamfering the first opening and the second opening.

This step may be performed by, for example, a drilling or milling device on the provided solid state body during processing to create the device model.

According to another exemplary embodiment of the invention, an engraving bit is used during the step of chamfering the first opening and the second opening. In other words, this exemplary embodiment may be seen as engraving the chamfer at the first opening and the second opening. In particular, an engraving bit can be a burin or also an automatically driven engraving tool. Consequently, this step can be carried out on a purely automatic basis, but also a manual and non-rotating engraving device may be used for such a method step.

Furthermore, the use of the cell culture device as described herein is an object of the present invention.

Thus, a cell culture device according to the present invention may be used for generating and cultivating uniform or substantially uniform cell aggregates. The uniform cell aggregates may also be referred to as three-dimensional uniform cell aggregates or spheroids.

Further, a cell culture device according to the present invention may be used in a method for substance and/or irradiation testing. Preferably, the method for substance testing is a screening method, more preferably a high-throughput screening method. Due to the design of the device, it is easily possible to use the device in experimental setups employing a high number of dilution series or replica.

Another major advantage of the invention is the fact that the device can be used in clonogenic cell survival assays. The device of the invention can be analyzed with digital image analysis which is a great advantage compared to the state of the art. Due to the spatially separated recessions it is possible to distinguish single clones, which is often difficult in the state of the art. Hence the analyses are more accurate, much faster and therefore also cheaper.

It is further an object of the present invention to provide a method of determining the influence of a substance and/or of irradiation on the growth of cells, wherein a device as disclosed herein is used in said method.

Thus, the present invention relates to an *in vitro* method of determining the influence of a substance on the growth of cells comprising at least the steps of:

a) Providing a device as disclosed herein;
b) Loading said device with cells;
c) Optionally incubating said device;
d) Adding said substance to said device comprising said cells;
e) Optionally incubating said device;
f) Determining the influence of said substance on said cells by determining the area or volume of individual cell aggregates in said device and/or the number of cells in individual cell aggregates in said device and comparing said area or volume and/or number to a reference.

The described in-vitro method can also relate to toxicological screening or drug/compound efficiacy testing.

It is preferred that the volume is determined via the projected area of individual cell aggregates. It is also preferred that the number of cells is determined via the volume. It is also preferred to determine the number of viable cells.

In a preferred embodiment of the method, said device is provided in step a) in a cell culture dish. In another preferred embodiment of the method, said cells, the device is loaded with in step b), are comprised in cell culture medium. In yet another preferred embodiment of the method, said device is incubated in steps c) and/or e) under cell culture conditions suitable for said cells. In another preferred embodiment of the method, said substance, the device is loaded with in step d), is comprised in cell culture medium. In another preferred embodiment, said reference area or volume and/or reference number is obtained from cells in a device not incubated with said substance. The device of the invention has the advantage that the testing areal is spatially separated from the cultivated cells. Therefore it is also possible to test substances which have not been specially prepared. If the substances do comprises relevant compounds these compounds can diffuses through the permeable hydrogel to the recessions with cells.

In still another preferred embodiment, said device is a device comprising an elongated recession element (200) as defined above (i.e. a device comprising an elongated recession element (200) for receiving a substance, wherein the first recession and the second recession are located in the device along a first line, wherein the elongated recession element expands in the device along a second line, and wherein the first line and the second line are not parallel to each other), and said substance is loaded into said elongated recession element.

In yet another preferred embodiment, said device is a device comprising an elongated recession element (200) as defined above (i.e. a device comprising an elongated recession element (200) for receiving cells, wherein the first recession and the second recession are located in the device along a first line, wherein the elongated recession element expands in the device along a second line, and wherein the first line and the second line are not parallel to each other), and said substance is released from a second type of cells, wherein the cells of the second type are loaded into said elongated recession element of said device.

The present invention also relates to an *in vitro* method of determining the influence of irradiation on the growth of cells comprising at least the steps of:

a) Providing a device as described above;
b) Loading said device with said cells;
c) Optionally incubating said device;
d) Irradiating said device;
e) Incubating said device;
f) Determining the influence of the irradiation on said cells by determining the area or volume of individual cell aggregates in said device and/or the number of cells in individual cell aggregates in said device
and comparing said area or volume and/or number to a reference.

It is preferred that the volume is determined via the projected area of individual cell aggregates. It is also preferred that the number of cells is determined via the volume. It is also preferred to determine the number of viable cells.

In a preferred embodiment of the method, said device is provided in step a) in a cell culture dish. In another preferred embodiment of the method, said cells, the device is loaded with in step b), are comprised in cell culture medium. In yet another preferred embodiment of the method, said device is incubated in steps c) and/or e) under cell culture conditions suitable for said cells. In another preferred embodiment, said reference area or volume and/or reference number is obtained from cells in a device not irradiated.

In another preferred embodiment relating to the above described methods, any optionally present cell culture medium is removed and viable cells or parts thereof are stained with a suitable compound. The person skilled in the art knows which compounds are suitable for a certain approach. It is especially preferred that viable cells are stained with a compound selected from 3-(4,5.Dimethylthiazol-2-yl)-2,5-diphenyltetrazoliumbromide (MTT) or Fluoresceine diacetate following step e) and prior to step f).

In still another preferred embodiment, the number of viable cells in individual cell aggregates is measured by staining viable cells with a compound selected from MTT, Fluoresceine diacetate, Propidium iodide and DAPI and by determining the fluorescence-intensity of the cell aggregates in at least one of the recessions of said device.

Cell aggregates generated and cultivated with the device according to the present invention thus represent a particularly suitable system for studying the characteristics of the particular type of cells used or the influence of specific parameters, such as e.g. potential cytotoxic agents or irradiation on the growth of the cells. Before this background, the present invention also refers to an *in vitro* method of determining the influence of a substance and/or irradiation on the growth of cells.

In an *in vitro* method according to the present invention, a device as defined above is provided. Preferably, the device is provided in a cell culture dish, wherein the device has been sterilized (e.g. by incubation in 70% ethanol for at least 4 h at 37°C), has been washed in phosphate-buffered saline (PBS) several times to remove the ethanol and has been placed into the dish, optionally comprising basal cell culture medium, under sterile conditions (e.g. using a laminar flow).

In the next step, the device is loaded with cells of interest, preferably by removing the buffer solution or the medium and then pipetting the cells in cell culture medium (such as e.g. DMEM or RPMI, optionally comprising additives and serum) onto said device comprised in the cell culture dish. The number of cells may be adapted to the intended size of the cell aggregates, e.g. by using between about 10,000 cells / ml to about 1,000,000 cells / ml. In principle, all types of cells may be used, even if the specific type of cells used fails to tend to form stabile spheroids. It can be particularly preferred to use cancerous cell lines or stem cells derived from humans.

The device is then incubated in order to generate uniform cell aggregates, preferably by incubating the dish comprising the device under standard cell culture conditions used for the type of cells (such as e.g. 37°C, 5% $CO_2$ for cell lines derived from humans). The incubation period can range from a few hours to several weeks.

In the next step, the device is then be loaded with the substance to be tested and/or the device is irradiated. In principle, any substance with a potential effect on the growth of cells may be used. It is of particular interest for the present invention to use potential cytotoxic agents. Thus, the influence of such cytotoxic agents on cancer cells may be determined using a method according to the present invention. Such substance may also be referred to as potential chemotherapeutic agents. Additionally or alternatively, the influence of irradiation on the cells may be determined. It can be of particular interest to employ irradiation types and doses as commonly used in radiation therapy, such as e.g. $^{137}$Cs in doses ranging from e.g. 5 to 30 Gy (see also examples 2 and 3).

The device is then again incubated, preferably under standard conditions as described above. The incubation period may range from a few hours to weeks, depending on the goal of the method.

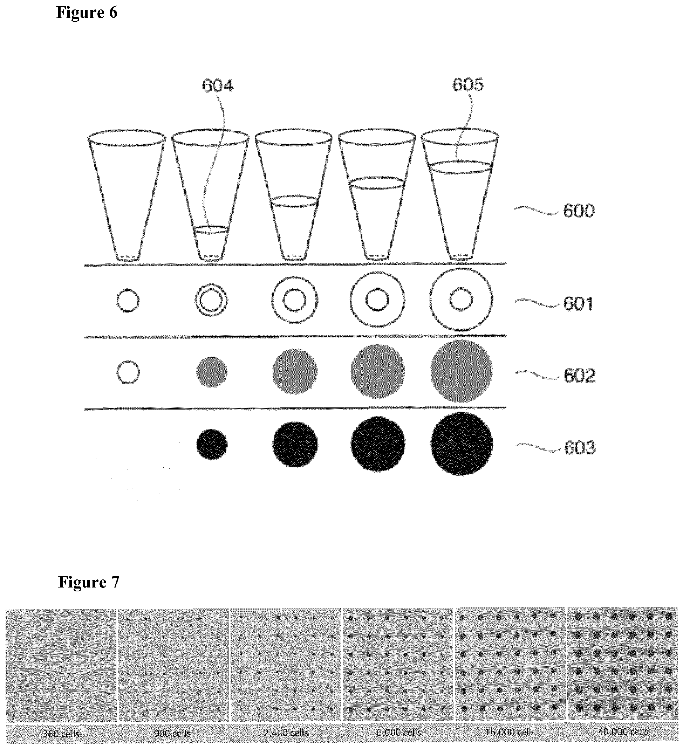

Optionally, viable cells are then stained, e.g. by MTT (3-(4,5.Dimethylthiazol-2-yl)-2,5-diphenyltetrazoliumbromid). This may be achieved by removing any medium present in the dish, adding medium with MTT and incubating for about 0.5 to 4 hours, followed by at least one washing step using e.g. PBS (see also Fig. 6).

In the next step, the area or the volume of individual cell aggregates in said device and/or the number of cells in individual cell aggregates in said device is determined in order to determine the influence of the substance and/or the irradiation on the cells. In order to determine the projected area, the device may be scanned (optionally in the dish) from the bottom using a conventional scanning device (see also example section). The area of the cell aggregates may then e.g. be determined using ImageJ from NIH or any conventional software for determining areas (see also example section).One may further determine the volume of the cell aggregate by taking into account the geometry of the recession (see above). If the number of viable cells should be determined, the viable cells in the cell aggregates are preferably stained with fluorescent dyes as set out above, the intensity of the fluorescence is determined and compared to a reference.

By comparing the area or volume and/or cell number to a reference, the influence of a substance and/or of the irradiation may be determined. The reference may either be a predetermined value (obtained e.g. from previous experiments employing identical cells in identical cell numbers without adding a substance or irradiating the cells) or may be derived directly from cells in a device not incubated with a substance or not irradiated.

In another preferred embodiment of the invention, the invention relates to a kit for generating and cultivating cell aggregates, comprising at least one of said devices multiwell plate comprising at least two wells wherein each well comprises one of said devices. The multiwell plate can be e.g. a 6-well plate, a 12-well plate or a 96-well plate. It is preferred that the device has the shape and the size of the well bottom.

It may be seen as a gist of the invention to provide for a microstructured device for generating and cultivating uniform or substantially uniform cell aggregates. The upper part of the recessions and the surface of the device are shaped to facilitate a transport of cells by gravity into at least one of the first recession and the second recession. Consequently, sedimentation of cells on surface is avoided due to the sloped surface, which preferably lacks any horizontal parts. Such device geometry can advantageously be provided by the production method as presented herein. The mechanical aspects of producing such a model, which are presented herein, allow for advantageous geometries of the device on a very small scale. Particularly in view of the embodiments described in the figures, a device for improved generation and cultivation of cell aggregates is provided, wherein an optical readout is also facilitated. Based on the created device model, a mould is generated, which is a negative form of the device to be produced. Furthermore, casting a solution into the mould leads to the desired microstructured device for generating and cultivating cell aggregates.

These and other features of the invention will become apparent from and elucidated with reference to the embodiments described hereinafter.

BRIEF DESCRIPTION OF THE DRAWINGS

Exemplary embodiments of the invention will be described in the following drawings.

Figs. 1 to 3 schematically show devices according to different exemplary embodiments of the invention.

Fig. 4 schematically shows a top view of the two devices shown in Fig. 3.

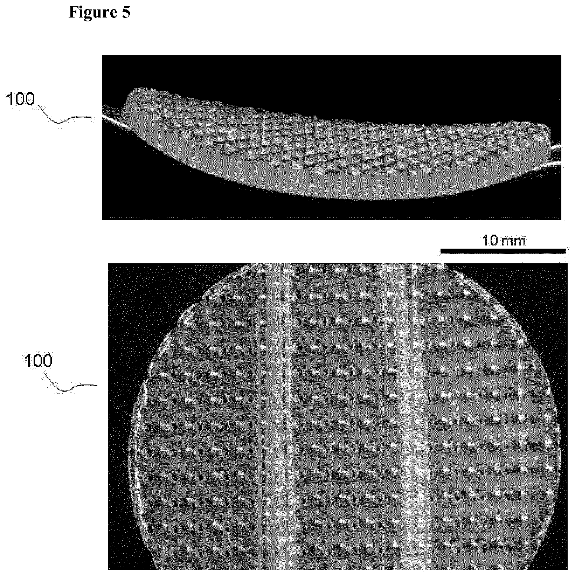

Fig. 5 shows a picture of a preferred device.

Fig. 6 schematically shows aspects of an optical detection used with a device shown in Fig. 3 according to an exemplary embodiment of the invention.

Fig. 7 shows a scan of the stained devices as described in Example 1.

Fig. 8 shows a software program for image analysis used in combination with devices according to the present invention as described in Example 1.

Fig. 9 shows a graph as a result of studies with a device according to the present invention as described in Example 1.

Fig. 10 shows a scan of the devices as described in Example 2.

Fig. 11 shows the volume of the cell aggregates depending on the irradiation dose used, see Example 2 for further details.

Fig. 12 shows a scan of the devices as described in Example 3.

Fig. 13 shows the volume of cell aggregates depending on the irradiation dose and the presence of cisplatinum, see Example 3 for further details.

Fig. 14 and 15 schematically show different devices according to different embodiments of the present invention.

Fig. 16 schematically shows a device according to an exemplary embodiment of the invention loaded with cells.

Fig. 17 schematically shows a device according to an exemplary embodiment of the invention.

Fig. 18 shows a flow diagram of a production method for generating a device according to an exemplary embodiment of the invention.

Fig. 19 a cutting process by means of which a brass body is processed to generate the device model according to an exemplary embodiment of the invention.

Fig. 20 shows a raster or grid of frustum of pyramids of the brass body of Fig. 19.

Fig. 21 shows a relief in a lead plate as a device model generated by means of the first solid state body of Fig. 19 according to an exemplary embodiment of the invention. A conically shaped punching tool is pressed into the lead plate.

Fig. 22 shows the device model of Fig. 19 with conical recessions and slanted or sloped partition walls..

Fig. 23 shows an enlarged image of the silicone mould, which is generated by the method as described with respect to Figs. 19 to 22 according to an exemplary embodiment of the invention.

In principle, identical parts are provided with the same reference symbols in the figures. Further, the figures are schematic and not in scale.

DETAILED DESCRIPTION OF THE INVENTION

In the following, embodiments of the present invention are described in more detail and with reference to the figures, if applicable.

Fig. 1 shows several cell culture devices 100 for generating and cultivating or cell aggregates 111, which comprises a first recession 101 for receiving cells and a second recession 102 for receiving cells. Furthermore, the device 100 comprises a partition wall 103, which separates the first and the second recession and a bottom part 113. As can be gathered from Fig. 1 a - c, the surface 112 of the device has a shape, which facilitates a transport of cells by gravity from a distal end of the device into at least one of the first recession and the second recessions.

Fig. 1 a and b show, that the surface can be shaped like a sloped sidewall 105 which causes the gravity transport effect. These side walls can end in a tip 107. But other shapes are also preferred, e.g. Fig. 3 c (left) shows a convex shape or a curved shape (right). These are also preferred embodiments of the invention. Consequently, the devices of Fig. 1 facilitate a high throughput uniform cell distribution within the device and advantageously avoid sedimentation of cells at undesired locations, e.g. the surface or recession side wall. As can be gathered from Fig. 1, the surface does not comprise any horizontal section or any horizontal portion, where the cells could disadvantageously sediment.

Fig. 1 d shows different possibilities for the bottom part 114 of the recessions. The upper part 106 is preferably always shaped as a truncated cone. The bottom part can have various geometries. E.g. the bottom part can just be flat like in Fig. 1 a - c. In this case the bottom part is the apical end of the upper part. Fig. 3 d shows a concave bottom part (second from the left) tapered bottom parts with different angles (two figures on the right).

The advantageous production method described herein allows for a production process in which the desired device with recessions having a diameter of a single cell can be produced by means of a corresponding device model and a corresponding mould. This provides the skilled person with novel possibilities of cell analysis.

Furthermore, the partition wall 103 provides for a section 106 between the distal end and the base section 104. Section 106 comprises a second slope (truncated cone) and it can be seen that the second slope is steeper than the slope of sidewall 105 of the surface 112. Therefore, the cell transport to the bottom of the device is provided. The device of Fig. 1 a comprises a partition wall with a tapered surface ending in a tip 107 at the distal end of the partition wall 103. Furthermore, the recession can comprise a tapered bottom part as shown in Fig 1 d (3. and 4. figure from the left). Providing such geometry of the device on the given scale of device 100 facilitates and improves the formation of cell aggregates, their cultivation as well as their analysis.

As can e.g. be gathered from Figs. 1 and 3, a raster of conical recessions microwells with respective chamfers at the opening, i.e. distal end, of the recessions, and a tapered bottom is presented. For example, the device model may be generated in a two-step process. After milling or drilling conical recessions, the recessions are chamfered by means of a milling or drilling tool. Thereby, the sloped sections 105a, 105b are generated at the corresponding device model by a milling or drilling tool. The depth of the conical recessions allows for an uncomplicated change of the cell culture medium, the transfer into another culture dish, and the staining of cell aggregates without flushing the aggregates out of the device.

According to another exemplary embodiment of the invention, Fig. 2 shows a cell culture device 100 which comprises a first recession 101 for receiving cells for generating and cultivating cell aggregates. Furthermore, the device 100 comprises an elongated recession element 200 for receiving a substance or cells. Another elongated recession element is depicted with element 201. Fig. 2 may be seen as a cross-section perpendicular to a line along which the first and the second recession of the device are positioned. The second recession may be seen from the bottom part of Fig. 2 and is depicted with 102. The top part of Fig. 2 is a cross-section through the first recession 101. The device 100 of Fig. 2 provides for a location of the first and the second recessions along a first line. Moreover, the elongated recession element 200 expands into the device along a second line wherein the first and the second lines are not parallel to each other. The top view of the bottom part of Fig. 2 depicts an inclined provision of the line along which the first and the second recessions 101, 102 are located compared to the elongated recession element 200. The conical recessions 101, 102 may have a diameter of a single cell which is approximately 15 micrometer. Nevertheless, also other diameter values are within the scope of the present invention. The use of the device 100 of Fig. 2 allows the determination of the influence of substances or the influence of a second cell type on the behaviour of cell aggregates such as spheroids. By means of the angle between the first and the second line, a concentration gradient can be generated along the diffusion path. Different angles between the first and the second line may be used in the devices of the present invention. Also, different substances can be filled in elongated recession elements 200 and 201 such that a different type of mixing of a first and a second substance via diffusion can be achieved. As can be gathered from Fig. 2, a plurality of recessions is provided as a row of conical recessions 202. Each recession comprises an upper part shaped like a truncated cone and the surface between the adjacently positioned recessions has a shape (no or substantially no horizontal parts) which facilitates the transport of cells by gravity into at least one of the adjacent recessions. Fig. 2 clearly shows that the surface comprises an inwardly sloped sidewall 105 which achieves such a gravity effect. Different scales or values regarding the opening angle of the chamfering shown in Fig. 2 at the first recession 101 can be applied. The different recessions are separated by bridges or bars of hydrogel material, like for example agarose, such that test substances may diffuse to the conical recessions 202. Due to the inclined and diagonal design in the row of recessions 202 and the elongated recession elements 201, 202, the desired gradient of concentration is achieved. For controlling the concentration gradient, the type and/or the concentration of the hydrogel as well as the angle between the elements can be adapted. For testing an invasive growth, the spheroid row can be filled with a degradable hydrogel, such as e.g. fibrin or matrigel.

According to another exemplary embodiment of the invention, a cell culture device 100 is depicted in Fig. 3. In this figure the base section 104 is shown. This device allows for a quantification of the volume of the cell aggregates without the need of removing the aggregates from the device; the optical readout is based on the shape of the recessions as truncated cones (also referred to as "conical shape"). This device can be preferably used with cells that do not form spheroids, such as e.g. most of the cells of well-established tumour cell lines. Fig. 3 shows two different geometries of the device 300. In particular, on the left hand side, a raster of 1x1 mm is shown (the distance between one tip of a partition wall (surface between two recessions) to the other tips is 1 mm in each direction), whereas the right hand side shows a 2x2 mm raster of the device (the distance between one tip of a partition wall to the other tips is 2 mm in each direction). In the device 100 of Fig. 3, the bottom 300 of the first recession and the bottom 301 of the second recession are flat. Furthermore, the first recession and the second recession have the shape of a truncated cone 302. The opening angle of the truncated cone may have different angles for different embodiments of such a device. Since the geometry of a truncated cone used in a device as described above is known (in particular, the radius at the bottom of the truncated cone and the angle between the axis and the slant is known), the volume of a cell aggregates in a recession may easily be determined if the diameter of a cell aggregates has been determined (e.g. by scanning the device from below in order to determine the area of the cell aggregates, i.e. from the bottom side, see also below and Figs. 6 to 11). Advantageously, the volume of a particular cell aggregates can thus be determined without the need of releasing the cell aggregates from the device. Furthermore, the geometry of the recessions facilitates the exchange of the cell culture medium including nutrients without unintentionally flushing the cells out of the device. Consequently, the device provides for a very practical and advantageous handling.

Fig. 3 also shows the base 104 of the device. Further, the figure shows that the partition wall 103 comprises an inwardly sloped sidewall 105a and an inwardly sloped sidewall 105b at the surface, resulting in the gravity-based transport of cells into the recessions. The slopes of the sidewalls 105a, 105b are not as steep as the upper part 106 of the recession. Conical recessions with a diameter of a single cell (about 15 $\mu$m) can be provided according to an embodiment similar to Fig. 3.

Fig. 4 is a schematic drawing of the device of Fig. 3 in a top view. The same reference numbers are used as in Fig. 3. Recession elements are also shown by reference numbers 302.

As can be gathered from Fig. 3, the depth of the recession may be about 2 mm. The depth may also be 1 mm, 1.2 mm, 1.24 mm, 1.26 mm, 1.28 mm, 2.0 mm, 2.2 mm, 2.5 mm, or any other desired appropriate value. Usually, a depth of the recession is used which is suitable to maintain the cell aggregates in the recession during any type of manipulation, e.g. when changing the medium or when staining the cell aggregates. In order to select the appropriate depth, the ratio of the depth to the diameter of the recession needs to be taken into account; thus, the minimal depth may preferably be in the range of 1.5 to 3 times the diameter of the recession. In this context the diameter of a recession is the diameter of the top of the truncated cone, which is wider than the bottom part. Depending on the diameter used, the depth may thus range from 0.02 mm to 3 mm. However, also other ratio between the depth and the diameter may be applied.

The production method allows for a huge variety of different shapes of the device as shown in Fig. 3. Furthermore, studies have shown that the device of Fig. 3 can be advantageously used for histological studies. Thus, the device 300 including the cell aggregates can be fixed, dehydrated, embedded in paraffin, and cut in a microtome. Consequently, the device of Fig. 3 provides for a practical improvement and increases the efficiency of cell aggregates analysis. If desired, a harvesting of the cell aggregates is facilitated by turning the device around and applying a centrifugal force. For example, 2 minutes of centrifugation at a rate of 200 g may be appropriate. However, also other values are possible. In order to provide for distances between the recessions without involving a horizontal or substantially horizontal surface, on which disadvantageously cells could sediment, recessions are provided in the upper region with an opening angle. Different values for said angle can be provided by the present invention. The choice of larger distances between the recessions facilitates cultivating of cells with high activity regarding mitosis and/or metabolic activity without creating supply-problems for the cells.

The amount of recessions per surface can be adapted to the requirements of the cells which are cultivated. However, it needs to be taken into account that a sufficient supply of the cells with medium and oxygen is provided until the end of the experiment. The larger the initial amount of cells per recession and the larger the proliferation, the larger the distance between the recessions should be. Also the metabolism of the cells used should be taken into account. In particular, tumour cells often show a strong tendency towards anaerobic glycolysis with a fast acidosis of the culture medium in case the cell density is too high. Such disadvantages can be avoided by means of adapting the geometry of the cell culture devise according to the present invention.

Table 1 depicts different geometric possibilities of forming the cell culture device (different rasters) together with the resulting number of recessions per $cm^2$ and the number of cells in each recession if 1 ml cell suspension at a concentration of $10^5$ cells / ml is used.

**Table 1**

| Raster [mm] | 0.2 x 0.2 | 0.316 x 0.316 | 0.5 x 0.5 | 1x1 | 2x2 | 3.316 x 3.316 | 5x5 |
|---|---|---|---|---|---|---|---|
| recessions/cm$^2$ | 2,500 | 1,000 | 400 | 100 | 25 | 10 | 4 |
| cells/recession at distributing 1 ml cell suspension/cm$^2$ with 100 cells/ml | 0.04 | 0.1 | 0.25 | 1 | 4 | 10 | 25 |
| cells/recession at distributing 1 ml cell suspension/cm$^2$ with 10$^5$ cells/ml | 40 | 100 | 250 | 1,000 | 4,000 | 10,000 | 25,000 |

Initially, the device is inserted into a culture dish, for example a multi-well plate or a Petri dish. After adding the cells in the form of a homogeneous cell suspension, the cells sediment into the recessions at the bottom, on which they form more or less coherent cell aggregates. This process can depend on the type of bottom part of the device. Consequently, different types of the bottom parts are described herein for the device. With a device according to the present invention, cell adhesion to the hydrogel does not happen. Further, the amount of cells in each recession is substantially the same. Depending on the volume of the particular cell aggregate in a recession, a corresponding cell level may form. Due to the conical shape, not only the height of the cell aggregates indicates the volume of said cell aggregates but also the maximal diameter of the cell aggregates; thus, the top level of the conical cell aggregates facing towards the opening of the recession can be used as such an indicator. The area of this top level can be microscopically read out by means of, for example, a scanner. A corresponding quantification can be performed subsequently. Consequently, the volume of the cell aggregate can be determined without having the need to release the cell aggregate from the device.

The top row of Fig. 6 depicts a side view 600 of five different filling levels in a conical shaped recession of a device according to the present invention. The top levels 604 and 605 can be optically read-out and allow for a high throughput and reliable read-out of the volume of the cell aggregates. Rows 601 to 603 show the conical recessions; 601 depicts an unstained situation, 602 depicts an MTT staining and 603 corresponds to a binaric presentation.

Fig. 14 shows another device for generating and cultivating cell aggregates 111 according to an exemplary embodiment of the invention. The device further comprises at least one breakthrough 1700. The partition wall 103 spatially separates the first and the second recession elements. Also the breakthrough 1701 is shown. Fig. 14 is a cross-section through the device 100, whereas Fig. 15 depicts the top view of the device 100 of Fig. 14. This perspective shows that the recessions are still connected via partition. It is preferred that the surface is shaped like a long edge. This embodiment of the invention may advantageously allow for a quantification of cellular invasion within a second, degradable hydrogel like for example fibrin or matrigel. The device 100 provides for a regular pattern of conical recessions with breakthroughs. A part of the loaded cells reaches the conical recessions, whereas the rest of the cells falls through the breakthrough 1700 and may be removed by placing the device 100 in another culture dish. In the next step, the device can be surrounded partially or also completely by a second hydrogel, such as e.g. fibrin. Consequently, the cell aggregates 111 may be completely surrounded by a second device or matrix. Depending on the potential of invasion of the cells, a migration of the latter can be observed in radial direction starting from the cell aggregates. The extent of the invasive growth can be surveyed and analysed via microscopy, histology or MTT-staining and scanning as already described above.

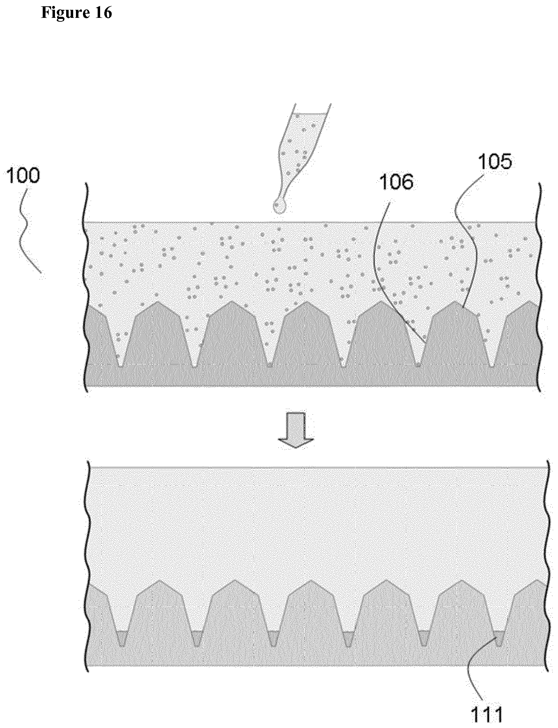

Fig. 16 shows another preferred embodiment of the invention. The device 100 is place inside a cell culture dish, e.g. a multiwell plate. The upper figure shows how the device 100 can be loaded with cells. In the bottom figure cell aggregates inside the recessions are shown.

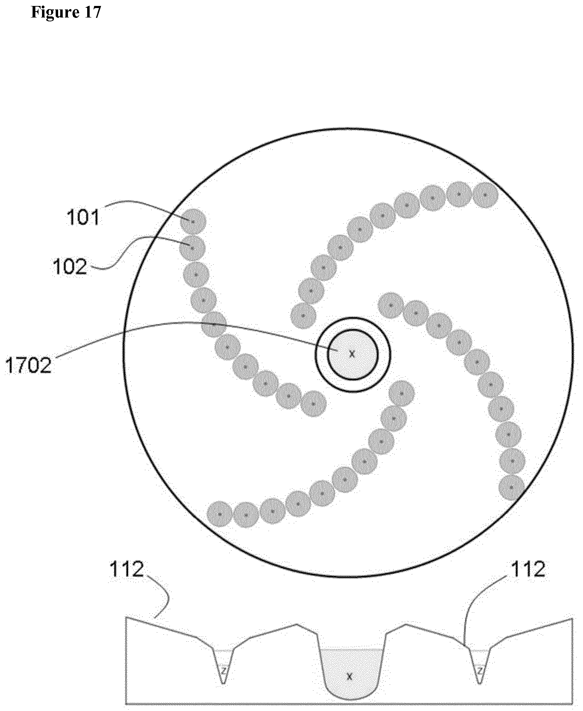

Fig. 17 shows another preferred embodiment of the invention. In this embodiment the device 100 further comprises a plurality of recessions shaped like the first 101 or the second recession 102 and an enlarged recession element 1702 for receiving a substance and/or cells. As shown in Fig. 17 the recessions are located in a way that at least two recessions have a different distance to the enlarged recession element.

Fig. 18 shows a flow diagram of a production method according to an exemplary embodiment of the invention. In particular, Fig. 18 shows a method of producing a device for generating and cultivating cell aggregates. The method comprises the step of providing for a solid state body S1 and the step of processing the solid state body S2, thereby creating the device model S3. Furthermore, generating a mould by moulding the device model S4 is shown. Also casting the device by filling the model with a solution S5 is depicted in Fig. 18. This general method of producing a device can be supplemented and varied in different ways. In particular, the step of processing can be machining like drilling or milling, electrical discharge machining, electro chemical machining but also non-cutting processes like pressing or punching are comprised in this method step. The mechanical step of processing the solid state body provides for certain partitions and advantages regarding the small size of the device, which cannot be achieved by other known procedures. For example, the advantageous geometry of having only angled distal ends of partition walls between the recessions in the device at a scale of 1 mm can precisely be provided by the presented method. However, with photolithographic or rapid prototyping or inkjet procedures, this is not possible. Releasing the device from the mould can easily be done by manually pulling the device from the mould. However, also a second or third centrifugation step may be applied.

Fig. 19 provides for another advantageous embodiment of the presented production method. In particular, the step of processing the solid state body S2 and thereby creating a device model S3 is specified in this embodiment. A miniature plane or slicer device 2100 is used in order to plane or slice wedge-shaped channels or grooves into the solid state body. In an exemplary embodiment, the solid state body may be out of brass. In particular, different distances may be used between the parallel channels or grooves. For example, a distance of 0.5 mm may be used but also other values are within the scope of the present invention. Subsequently, additional channels or grooves are generated perpendicular to the first parallel channels or grooves. The first and second grooves may also extend in non perpendicular directions to each other. Again, the same tool 2100 is used. The plane or slicer tool 2100 may be moved automatically or manually. The first parallel channels or grooves are depicted with 2101 and the perpendicular rows of channels are depicted with 2102.

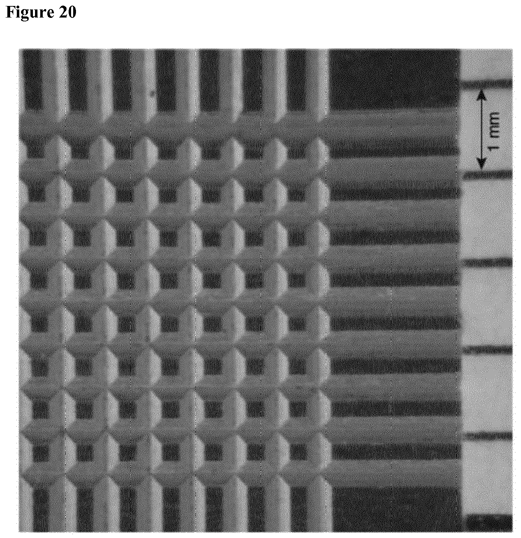

Fig. 20 shows that a grid of frustum of pyramids is generated by the procedure as described with respect to Fig. 19. Furthermore, the solid state body which has been processed so far can be pressed into another body by, for example, a hydraulic press such that the structure and relief shown in Fig. 20 is pressed into said second solid state body. Such second solid state body may be of ductile material like e.g. lead. Consequently, a relief with quadratic recessions with angled separation walls is generated.

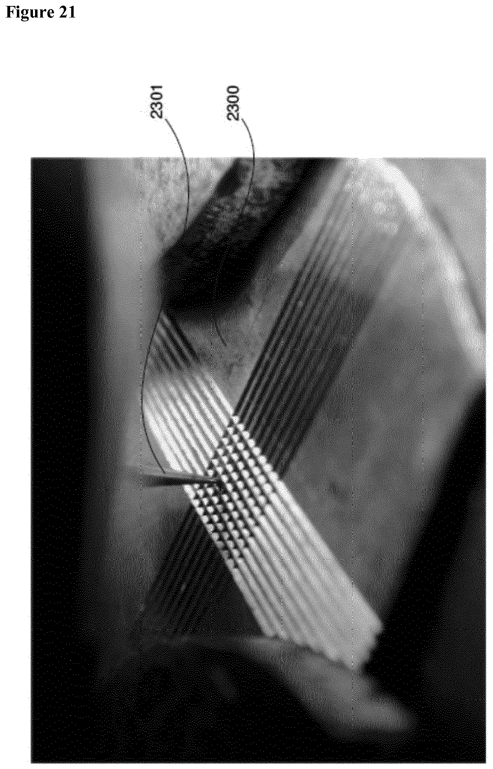

Fig. 21 shows an engraving bit like a burin or an automatically driven engraving tool 2301 to generate conical recessions by pressing tool 2301 into the second solid state body 2300. For example, a whetted mandrill or thorn may be used in order to push into the material of the second solid state body. When performing the steps described for figures 19 to 21 the user is provided with a respective device model.

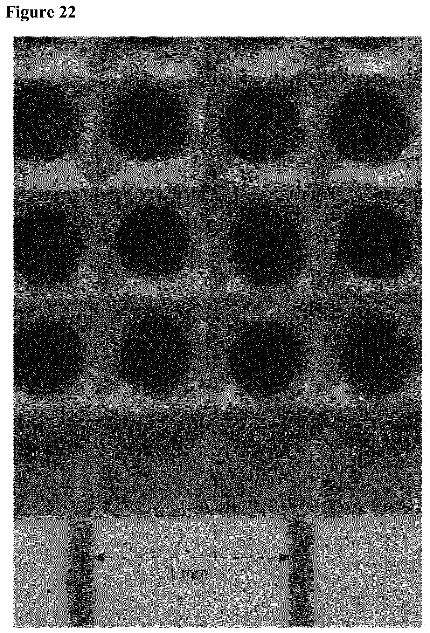

As can be gathered from Fig. 22, a device model with conical recessions and partition walls is generated wherein the partition walls have a shape facilitating the transport of the cells by gravity from a distal end of the partition wall into the recessions of the device to be generated out of the provided device model. Thus, Figs. 19 to 22 show how the steps of providing for a solid state body S1,

processing the solid state body S2 and thereby creating a device model S3, as described in Fig. 18, can be embodied.

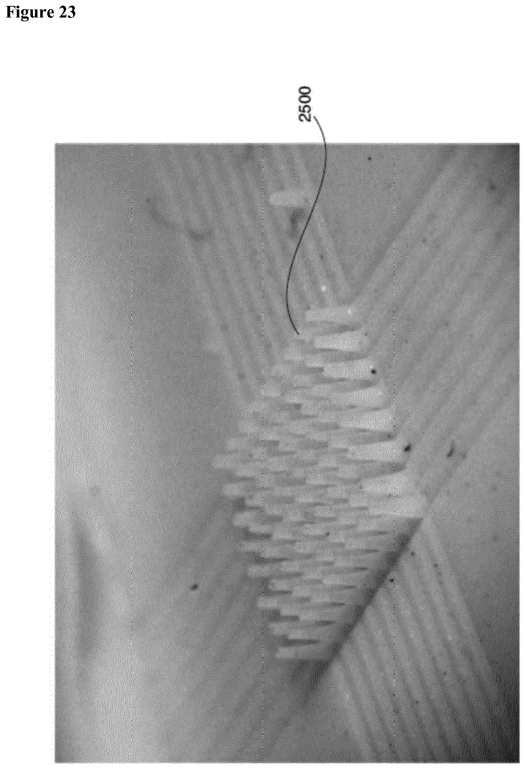

Fig. 23 shows a mould which has been generated, in accordance with step S4, with a device model as described in detail with respect to Figs. 19 to 22. Based on the mould 2500, a device can be produced by casting the device by filling the mould 2500 with a solution such as e.g. an agarose solution, in a high throughput and commercial manner.

The following advantages shall be noted for the production method as described with respect to Figs. 19 to 23: the production method may have the advantage that a further miniaturisation is possible compared to the use of rotating drilling or milling devices. In particular, the embodiment of the production method provides for the generation of conical recessions with a diameter of a single cell which is approximately 15 micrometer. Clearly, such a small conical recession is of interest in biological assays for cells and cannot be produced by drilling or milling. Consequently, the embodiment presented in Figs. 19 to 23 provides for an improved device for studying cells.

**EXAMPLES**

Example 1: Serial dilution of cells in a cell culture device with conical recessions

1. a 2.4 % agarose solution was prepared and heated in a microwave oven until boiling;
2. a pair of silicone moulds was pre-warmed to about 80°C on a heat plate and filled with agarose solution;
3. in order to remove bubbles, the silicone moulds were centrifuged shortly (e.g. 1 min. at 2,000g);
4. a glass plate was placed into each mould to provide a plane surface of the devices;
5. moulds were cooled to allow gelling of the agarose;
6. after removing from the moulds, round discs of 22 mm diameter were punched out of the device, so that each device covers the bottom of a well of a 12-well plate (area 3.8 cm$^2$);
7. devices were sterilized in 70% ethanol for 4 hrs. at 37°C;
8. devices were washed in sterile PBS for 1 hour on a shaker. Washing was repeated 4 times to remove the ethanol;
9. devices were placed in a 12 well plate with 1 ml of PBS in each well and centrifuged shortly (1 min. at 2,000g) in order to remove bubbles;
10. PBS was removed using a Pasteur pipette and replaced with 3.8 ml of cell suspension according to the table depicted below:

| device # | 1 | 2 | 3 | 4 | 5 | 6 |
|---|---|---|---|---|---|---|
| **Cells/ml** | 9,000 | 22,500 | 60,000 | 160,000 | 400,000 | 1,000,000 |
| **Cells/recession** | 360 | 900 | 2,400 | 6,000 | 16,000 | 40,000 |

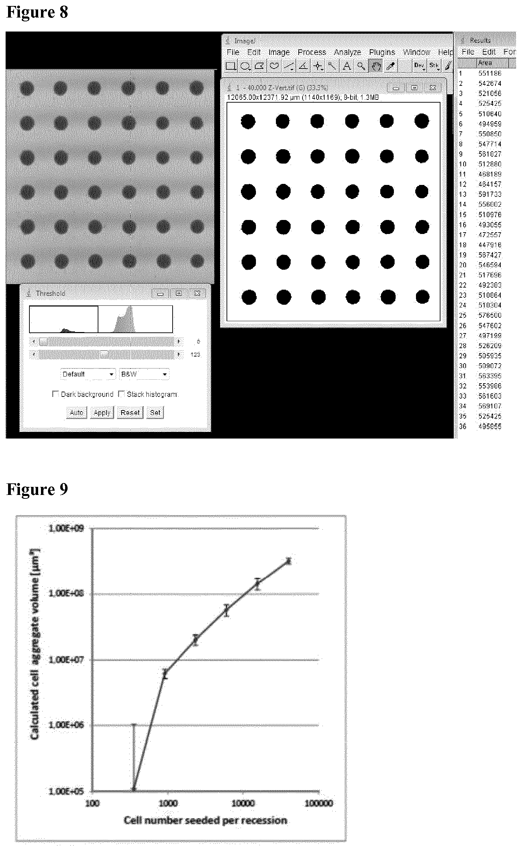

11. after sedimentation of cells, the devices were incubated each with 1 ml of unsupplemented DMEM containing 0.5 mg/ml of MTT (3-(4,5-Dimethylthiazol-2-yl)-2,5-diphenyltetrazoliumbromid) at 37°C for 4 hrs;
12. stained devices were then washed in PBS 3 times and scanned using a high-resolution office scanner;
13. from the central portion of each scan, a clipping comprising 36 cell aggregates was selected and stored as a greyscale TIF image. These images are shown in Figure 7;
14. the area A of each cell aggregate was measured using ImageJ image analysis software (see Figure 8);
15. from the resulting values, volume V of each cell aggregate was calculated using the formula depicted below

$$R = \sqrt{\frac{A}{\pi}}$$

$$h = \frac{R - r}{\tan \varphi}$$

$$V = \frac{h \cdot \pi}{3} \cdot (R^2 + R \cdot r + r^2)$$

Known variables: Radius r = 111 μm Angle $\varphi$= 12.5°

16. the resulting volumes were plotted as a graph, showing a linear correlation between loaded cell numbers and measured volumes (see Figure 9).

Example 2: Dose-response curve of irradiated tumour cell aggregates

1. Agarose cell culture device with conical recessions and a raster of 2x2 mm were prepared according to steps 1 to 5 as described in Example 1;

2. after removing from the moulds, round discs of 34 mm diameter were punched out of the device, so that each device covers the bottom of a well of a 6-well plate (area 9.5 cm$^2$) and comprises about 230 identical recessions;

3. devices were sterilized in 70% ethanol for 4 hrs. at 37°C;

4. devices were washed in sterile PBS for 1 hour on a shaker. Washing was repeated 4 times to remove the ethanol;

5. devices were placed in a 6-well plate with each 2 ml unsupplemented cell culture medium and centrifuged shortly (1 min. at 2,000g) in order to remove bubbles;

6. MTPa mammary carcinoma cells were released from cell cultures flasks by trypsinisation;

7. medium on top of each device was removed using a Pasteur pipette and replaced with 9.5 ml of cell suspension with 25,000 cells/ml, resulting in approximately 238,000 cells per well;

8. plates were incubated at 37°C, 5% $CO_2$ and 100% humidity so that cells were allowed to sediment into the devices and to form cell aggregates, resulting in an initial density of approximately 1,000 cells per aggregate;

9. after an incubation period of 48 hrs., devices with cells were irradiated at a dose rate of 60 cGy/min. in a $^{137}$Cs device;

10. cultivation was continued for a total of 16 days with culture medium changes every three days;

11. for evaluation of treatment effect on cell aggregate volumes, devices were incubated with each 2 ml of unsupplemented DMEM containing 0.5 mg/ml of MTT at 37°C for 4 hrs;

12. stained devices were washed in PBS three times and scanned at 1600 dpi using a high-resolution office scanner;

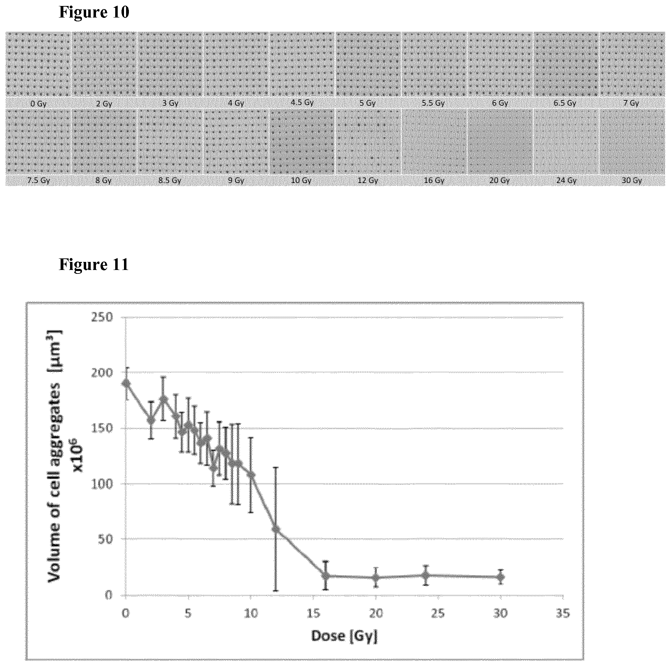

13. from the central portion of each scan, a clipping comprising 100 cell aggregates was selected and stored as a greyscale TIF image, as depicted in Figure 10;

14. the area of each cell aggregate was measured using ImageJ image analysis software as described above;

15. calculated cell aggregate volumes were plotted against irradiation dose, see Figure 11.

Example 3: Chemoradiation of tumour cell aggregates in a cell culture device with conical recessions

1. agarose devices fitting 6-well-plates were prepared according to steps 1 to 5 as described in Example 2;

2. devices were placed in a 6-well plate, each with 2 ml unsupplemented cell culture medium and centrifuged shortly (1 min. at 2,000g) in order to remove bubbles;

3. MiaPaCa-2 pancreatic carcinoma cells were released from cell cultures flasks by trypsinisation;

4. medium on top of each device was removed using a Pasteur pipette and replaced with 9.5 ml of cell suspension with 25,000 cells/ml, resulting in approximately 238,000 cells per well;

5. plates were incubated at 37°C, 5% $CO_2$ and saturated humidity so that cells were allowed to sediment into the matrices and to form cell aggregates, resulting in an initial density of approximately 1,000 cells per aggregate;

6. the next day, devices with cell aggregates were transferred into new wells with medium containing either 10 μmol of cisplatinum or medium with an identical quantity of NaCl as control;

7. after an incubation period of 10 hrs., devices with cells were irradiated at a dose rate of 60 cGy/min. using $^{137}$Cs;

8. irradiated devices with cells were incubated another 8 hours and then transferred into new plates with fresh medium; cultivation was continued for a total of 16 days with culture medium changes every three days;

9. for evaluation of treatment effect on cell aggregate volumes, devices were incubated with each 2 ml of unsupplemented DMEM containing 0.5 mg/ml of MTT at 37°C for 4 hours;

10. stained devices were then washed in PBS 3 times and scanned at 1600 dpi using a high-resolution office scanner;

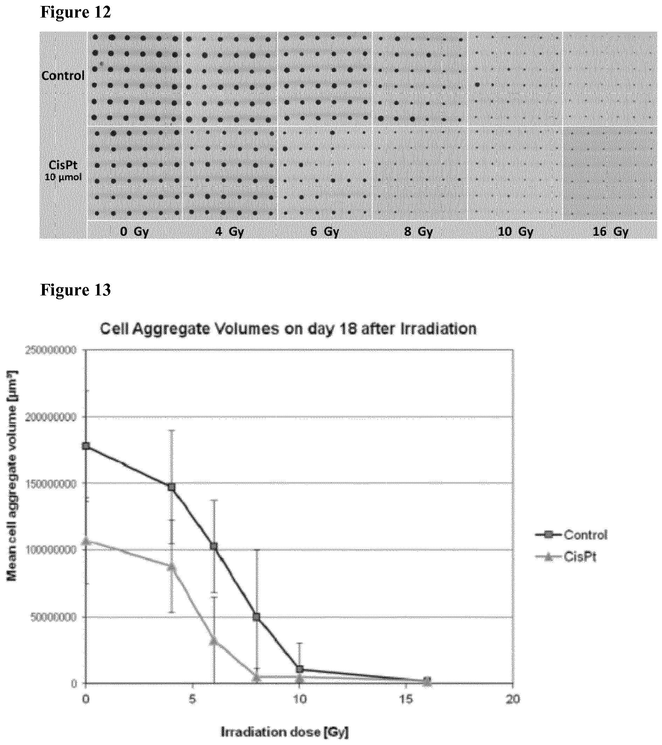

11. from the central portion of each scan, a clipping comprising 36 cell aggregates was selected and stored as a greyscale TIF image, as depicted in Figure 12;

12. the area of each cell aggregate was measured using ImageJ image analysis software as described above;
13. calculated cell aggregate volumes were plotted as a graph. The activity of cisplatinum as a radiosensitising agent can be derived from Figure 13.

**References**

(1) Tung Y-C et al. (2011) High-throughput 3D spheroid culture and drug testing using a 384 hanging drop array.

(2) Friedrich J et al. (2009) Spheroid-based drug screen: considerations and practical approach. Nature Protocols 4: 309-324

(3) Ivascu A et al. (2006) Rapid Generation of Single-Tumor Spheroids for High-Throughput Cell Function and Toxicity Analysis. J Biomol Screen 11: 922-932

(4) Tang MD et al. (2003) Molding of Three-Dimensional Microstructures of Gels. J. AM. CHEM. SOC. 125: 12988-12989

(5) Fukuda J et al. (2005) Orderly Arrangement of Hepatocyte Spheroids on a Microfabricated Chip. Tissue Engineering 11: 1254-1262

(6) Napolitano AP et al. (2007) Scaffold-free three-dimensional cell culture utilizing micromolded nonadhesive hydrogels. BioTechniques 43:494-500

**Claims**

1. Cell culture device (100) for generating and cultivating cell aggregates (111), the device comprising,

   • a cell repellent hydrogel substrate,
   • a surface (112),
   • at least a first (101) and a second recession (102) for receiving cells for generating and cultivating cell aggregates,

   wherein the first (101) and the second recessions (102) are spatially separated, and wherein the surface (112) between the first (101) and the second recession (102) is a sloped surface and
   wherein the first (101) and the second recessions (102) comprise an upper part (106) and a bottom part (114) and wherein the upper part (106) has the shape of an truncated cone (302) with an opening angle of 15 to 30° and the ratio of the depth of the upper part (106) to the diameter of the widest end of the upper part (106) is at least 1.5 : 1.

2. Device (100) according to claim 1, wherein the surface (112) between the first (101) and the second recession (102) does not comprise a horizontal or substantially horizontal part and/or wherein the surface (112) between the first (101) and the second recession (102) is a curved and/or convex surface (112).

3. Device (100) according to claim 1 or 2, wherein the surface (112) comprises at least one sloped side wall (105) and wherein the truncated cone (302) of the upper part (106) of at least one recession is steeper than the sloped side wall (105) and/or wherein the bottom part (114) of at least one recession comprises a tapered bottom, a concave bottom or a flat bottom..

4. Device (100) according to at least one of the preceding claims, wherein the device (100) is made of agarose, alginate or chemical modifications or a mixture thereof.

5. Device (100) according to at least one of the preceding claims, further comprising a plurality of recessions shaped like the first (101) or the second recession (102) and at least one enlarged recession element (1702) for receiving a substance and/or cells,
   wherein

the recessions are located in a way that at least two recessions have a different distance to the enlarged recession element.

**6.** Device (100) according to claim 8, wherein the enlarged recession element is an elongated recession element (200), preferred embodied as a channel.

**7.** Device (100) according to claim 9 wherein at least the first recession (101) and the second recession (102) are located in the device (100) along a first line, wherein the elongated recession element expands in the device along a second line, and wherein the first line and the second line are not parallel to each other.

**8.** Device (100) according to claim 10, wherein the plurality of recessions are arranged as one or more rows or concentric circles of recessions (202), and wherein the first recession and the second recession are part of the one or more rows or circles of recessions.

**9.** Device (100) according to one of claims 3 to 11, wherein the sloped side wall (105) has an angle which is selected from the group of angle ranges comprising 20-70°, preferred 30-60°, more preferred 35-55°, and most preferred 40-50°.

**10.** Method of producing a device (100) according to at least one of the preceding claims, the method comprising the steps of

a) providing for a solid state body,
b) processing the solid state body thereby creating a device model, the processing comprising machining the first recession and the second recession into the solid state body, preferred carried out by drilling, milling, electrical discharge machining, electro chemical machining or a non-cutting method preferred pressing, punching, embossing or 3D Printing
c) generating a mould by moulding the device model, and
d) casting the device by filling the mould with a solution comprising a cell repellent hydrogel,
e) centrifuging the mould with the solution.

**11.** Use of a device (100) according to one of claims 1 to 9 for generating and cultivating cell aggregates, preferred in a method for substance and/or irradiation testing on cells.

**12.** Use according to claim 11 comprising at least the steps of:

a) Providing a device (100) according to one of claims 1 to 9;
b) Loading said device (100) with said cells;
c) Optionally incubating said device (100);
d) Adding said substance to said device (100) comprising said cells;
e) Optionally incubating said device (100);
f) Determining the influence of said substance on said cells by determining the area or volume of individual cell aggregates in said device (100) and/or the number of cells in individual cell aggregates in said device (100) and comparing said area or volume and/or number to a reference.

**13.** Use according to claim 11 or 12, wherein said device (100) is a device (100) according to one of claims 5 to 8 and wherein said substance is loaded into said enlarged recession element of said device (100) and preferred wherein said substance is released from a second type of cells, wherein the cells of the second type are loaded into said enlarged recession element of said device (100).

**14.** Use according to claim 11 comprising at least the steps of:

a) Providing a device (100) according to one of claims 1 to 9;
b) Loading said device (100) with said cells;
c) Optionally incubating said device (100);
d) Irradiating said device (100);
e) Optionally incubating said device (100);
f) Determining the influence of the irradiation on said cells by determining the area or volume of individual cell aggregates in said device (100) and/or the number of cells in individual cell aggregates in said device (100) and comparing said area or volume and/or number to a reference.

15. Kit for generating and cultivating cell aggregates (111), comprising at least one device (100) according to at least one of the claims 1 to 9 and a multiwell plate comprising at least two wells wherein each well comprises one of said devices (100).

**Patentansprüche**

1. Zellkulturvorrichtung (100) zur Erzeugung und Kultivierung von Zellaggregaten (111), wobei die Vorrichtung umfasst:

   • ein zellabweisendes Hydrogelsubstrat,
   • eine Oberfläche (112),
   • wenigstens eine erste (101) und eine zweite Vertiefung (102) zur Aufnahme von Zellen zur Erzeugung und Kultivierung von Zellaggregaten,

   wobei die ersten (101) und die zweiten (102) Vertiefungen räumlich getrennt sind, und wobei die Oberfläche (112) zwischen der ersten (101) und der zweiten (102) Vertiefung eine geneigte Oberfläche ist, und
   wobei die ersten (101) und die zweiten (102) Vertiefungen einen Oberteil (106) und ein Unterteil (114) umfassen, und wobei der Oberteil (106) die Form eines Kegelstumpfes (302) mit einem Öffnungswinkel von 15 bis 30° aufweist und das Verhältnis der Tiefe des Oberteils (106) zu dem Durchmesser des breitesten Endes des Oberteils (106) wenigstens 1,5 : 1 beträgt.

2. Vorrichtung (100) nach Anspruch 1, wobei die Oberfläche (112) zwischen der ersten (101) und der zweiten Vertiefung (102) keinen horizontalen oder im Wesentlichen horizontalen Teil umfasst, und/oder wobei die Oberfläche (112) zwischen der ersten (101) und der zweiten Vertiefung (102) eine gekrümmte und/oder konvexe Oberfläche (112) ist.

3. Vorrichtung (100) nach Anspruch 1 oder 2, wobei die Oberfläche (112) wenigstens eine geneigte Seitenwand (105) umfasst, und wobei der Kegelstumpf (302) des Oberteils (106) von wenigstens einer Vertiefung steiler ist als die geneigte Seitenwand (105), und/oder wobei das Unterteil (114) von wenigstens einer Vertiefung einen kegelförmigen Boden, einen konkaven Boden oder einen flachen Boden umfasst.

4. Vorrichtung (100) nach einem der vorhergehenden Ansprüche, wobei die Vorrichtung (100) aus Agarose, Alginat, chemischen Modifikationen davon oder einem Gemisch daraus besteht.

5. Vorrichtung (100) nach einem der vorhergehenden Ansprüche, die ferner eine Vielzahl von Vertiefungen umfasst, die wie die erste (101) oder die zweite Vertiefung (102) geformt sind, und wenigstens ein vergrößertes Vertiefungselement (1702) zum Aufnehmen von einer Substanz
und/oder Zellen,
wobei
die Vertiefungen so angeordnet sind, dass wenigstens zwei Vertiefungen einen unterschiedlichen Abstand zu dem vergrößerten Vertiefungselement haben.

6. Vorrichtung (100) nach Anspruch 8, wobei das vergrößerte Vertiefungselement ein verlängertes Vertiefungselement (200) ist, das bevorzugt als Kanal ausgebildet ist.

7. Vorrichtung (100) nach Anspruch 9, wobei wenigstens die erste Vertiefung (101) und die zweite Vertiefung (102) entlang einer ersten Linie in der Vorrichtung (100) angeordnet sind,
wobei sich das verlängerte Vertiefungselement in der Vorrichtung entlang einer zweiten Linie erstreckt, und
wobei die erste Linie und die zweite Linie nicht parallel zueinander sind.

8. Vorrichtung (100) nach Anspruch 10,
wobei die Vielzahl von Vertiefungen als eine oder mehrere Reihen oder konzentrische Kreise von Vertiefungen (202) angeordnet sind, und wobei die erste Vertiefung und die zweite Vertiefung Teil der einen oder mehreren Reihen oder Kreise von Vertiefungen sind.

9. Vorrichtung (100) nach einem der Ansprüche 3 bis 11, wobei die geneigte Seitenwand (105) einen Winkel aufweist, der aus der Gruppe von Winkelbereichen ausgewählt ist, die 20-70°, bevorzugt 30-60°, stärker bevorzugt 35-55°, und am stärksten bevorzugt 40-50° umfasst.

10. Verfahren zur Herstellung einer Vorrichtung (100) nach wenigstens einem der vorhergehenden Ansprüche, das die folgenden Schritte umfasst:

   a) Bereitstellen eines Festkörpers,
   b) Bearbeiten des Festkörpers und dadurch Erzeugen eines Vorrichtungsmodells, wobei das Bearbeiten das maschinelle Herstellen der ersten Vertiefung und der zweiten Vertiefung in dem Festkörper umfasst, das bevorzugt durch Bohren, Fräsen, Funkenerodieren, elektrochemische Bearbeitung oder eine spanlose Bearbeitung, bevorzugt Pressung, Stanzen, Prägung oder 3D-Drucken, ausgeführt wird,
   c) Erzeugen einer Gussform durch Formen des Vorrichtungsmodells, und
   d) Gießen der Vorrichtung durch Füllen der Gussform mit einer Lösung, die ein zellabweisendes Hydrogel umfasst,
   e) Zentrifugieren der Gussform mit der Lösung.

11. Verwendung einer Vorrichtung (100) nach einem der Ansprüche 1 bis 9 zur Erzeugung und Kultivierung von Zellaggregaten, bevorzugt in einem Verfahren zur Substanz- und Strahlungstestung auf Zellen.

12. Verwendung nach Anspruch 11, das wenigstens die folgenden Schritte umfasst:

   a) Bereitstellen einer Vorrichtung (100) nach einem der Ansprüche 1 bis 9;
   b) Beladen der Vorrichtung (100) mit den Zellen;
   c) Wahlweise Inkubieren der Vorrichtung (100);
   d) Zugeben der Substanz zu der Vorrichtung (100), welche die Zellen umfasst;
   e) Wahlweise Inkubieren der Vorrichtung (100);
   f) Bestimmen des Einflusses der Substanz auf die Zellen durch Bestimmen der Fläche oder des Volumens einzelner Zellaggregate in der Vorrichtung (100) und/oder der Anzahl von Zellen in einzelnen Zellaggregaten in der Vorrichtung (100) und Vergleichen der Fläche oder des Volumens und/oder der Anzahl mit einer Referenz.

13. Verwendung nach Anspruch 11 oder 12, wobei es sich bei der Vorrichtung (100) um eine Vorrichtung (100) nach einem der Ansprüche 5 bis 8 handelt, und wobei die Substanz in das vergrößerte Vertiefungselement der Vorrichtung (100) geladen wird, und wobei bevorzugt die Substanz aus einem zweiten Zelltyp freigesetzt wird, wobei die Zellen des zweiten Zelltyps in das vergrößerte Vertiefungselement der Vorrichtung (100) geladen werden.

14. Verwendung nach Anspruch 11, die wenigstens die folgenden Schritte umfasst:

   a) Bereitstellen einer Vorrichtung (100) nach einem der Ansprüche 1 bis 9;
   b) Beladen der Vorrichtung (100) mit den Zellen;
   c) Wahlweise Inkubieren der Vorrichtung (100);
   d) Bestrahlen der Vorrichtung (100);
   e) Bestimmen des Einflusses der Bestrahlung auf die Zellen durch Bestimmen der Fläche oder des Volumens einzelner Zellaggregate in der Vorrichtung (100) und/oder der Anzahl von Zellen in einzelnen Zellaggregaten in der Vorrichtung (100) und Vergleichen der Fläche oder des Volumens und/oder der Anzahl mit einer Referenz.

15. Kit zur Erzeugung und Kultivierung von Zellaggregaten (111), das wenigstens eine Vorrichtung (100) nach einem der Ansprüche 1 bis 9 und eine Mikrotiterplatte umfasst, die wenigstens zwei Vertiefungen umfasst, wobei jede Vertiefung eine der Vorrichtungen (100) umfasst.

**Revendications**

1. Dispositif (100) de culture de cellules destiné à produire et cultiver des agrégats (111) de cellules, le dispositif comprenant :

   - un substrat d'hydrogel répulsif vis-à-vis des cellules,
   - une surface (112),

au moins un premier creux (101) et un deuxième creux (102) destinés à recevoir des cellules en vue de produire et de cultiver des agrégats de cellules,

le premier creux (101) et le deuxième creux (102) étant spatialement séparés, la surface (112) présente entre le premier creux (101) et le deuxième creux (102) étant une surface en pente,

le premier creux (101) et le deuxième creux (102) comprenant une partie supérieure (106) et une partie de fond (114) et

la partie supérieure (106) présentant la forme d'un tronc de cône (302) dont l'angle d'ouverture est de 15 à 30°, le rapport entre la profondeur de la partie supérieure (106) et le diamètre de l'extrémité la plus large de la partie supérieure (106) étant d'au moins 1,5:1.

2. Dispositif (100) selon la revendication 1, dans lequel entre le premier creux (101) et le deuxième creux (102), la surface (112) ne comprend pas de partie horizontale ou essentiellement horizontale et/ou dans lequel la surface (112) située entre le premier creux (101) et le deuxième creux (102) est une surface (112) incurvée et/ou convexe.

3. Dispositif (100) selon les revendications 1 ou 2, dans lequel la surface (112) comprend au moins une paroi latérale (105) en pente et dans lequel le tronc de cône (302) de la partie supérieure (106) au moins un creux est en pente plus forte que la paroi latérale (105) en pente et/ou dans lequel la partie de fond (114) d'au moins un creux comprend un fond effilé, un fond concave ou un fond plat.

4. Dispositif (100) selon au moins l'une des revendications précédentes, dans lequel le dispositif (100) est réalisé en agarose, en alginate, en modifications chimiques ou en un de leurs mélanges.

5. Dispositif (100) selon au moins l'une des revendications précédentes, comprenant en outre plusieurs creux configurés de la même manière que le premier creux (101) ou le deuxième creux (102) et au moins un élément (1702) en creux agrandi qui reçoit une substance et/ou des cellules, les creux étant disposés de telle sorte qu'au moins deux creux présentent des distances différentes par rapport à l'élément en creux agrandi.

6. Dispositif (100) selon la revendication 8, dans lequel l'élément en creux allongé est un élément (200) en creux agrandi de préférence configuré en canal.

7. Dispositif (100) selon la revendication 9, dans lequel au moins le premier creux (101) et le deuxième creux (102) sont disposés dans le dispositif (100) le long d'une première ligne, l'élément en creux allongé s'étendant dans le dispositif suivant une deuxième ligne, la première et la deuxième ligne n'étant pas parallèles l'une à l'autre.

8. Dispositif (100) selon la revendication 10, dans lequel les différents creux sont agencés sous la forme d'une ou plusieurs rangées ou cercles concentriques de creux (202) et dans lequel le premier creux et le deuxième creux font partie de la ou des rangées ou cercles de creux.

9. Dispositif (100) selon l'une des revendications 3 à 11, dans lequel l'angle de la paroi latérale (105) en pente est sélectionné dans l'ensemble des angles compris entre 20 et 70°, de préférence entre 30 et 60°, de manière plus préférable entre 35 et 55° et de la manière la plus préférable entre 40 et 50°.

10. Procédé de production d'un dispositif (100) selon au moins l'une des revendications précédentes, le procédé comportant les étapes qui consistent à :

   a) prévoir un corps à l'état solide,
   b) traiter le corps à l'état solide pour ainsi former un modèle de dispositif, le traitement comprenant l'usinage du premier creux et du deuxième creux dans le corps à l'état solide, de préférence par forage, meulage, usinage par décharges électriques, usinage électrochimique ou un procédé autre que de découpe, de préférence pressage, perforation, gaufrage ou impression en 3 dimensions,
   c) produire un moule en moulant le modèle de dispositif et
   d) mouler le dispositif en remplissant le moule d'une solution comprenant un hydrogel répulsif vis-à-vis des cellules, et
   e) centrifuger le moule contenant la solution.

11. Utilisation d'un dispositif (100) selon l'une des revendications 1 à 9 pour produire et cultiver des agrégats de cellules, de préférence dans un procédé de test de substances et/ou d'irradiation de cellules.

**12.** Utilisation selon la revendication 11, comprenant au moins les étapes qui consistent à :

> a) prévoir un dispositif (100) selon l'une des revendications 1 à 9,
> b) charger des cellules dans ledit dispositif (100),
> c) facultativement, incuber ledit dispositif (100),
> d) ajouter ladite substance audit dispositif (100) comprenant lesdites cellules,
> e) facultativement, incuber ledit dispositif (100),
> f) déterminer l'influence de ladite substance sur lesdites cellules en déterminant la superficie ou le volume d'agrégats individuels de cellules dans ledit dispositif (100) et/ou le nombre de cellules dans les agrégats individuels de cellules présents dans ledit dispositif (100), et comparer ladite superficie, ledit volume et/ou ledit nombre à une référence.

**13.** Utilisation selon les revendications 11 ou 12, dans laquelle ledit dispositif (100) est un dispositif (100) selon l'une des revendications 5 à 8, dans lequel ladite substance est chargée dans ledit élément en creux agrandi dudit dispositif (100) et de préférence dans lequel ladite substance est libérée par un deuxième type de cellules, les cellules du deuxième type étant chargées dans ledit élément en creux agrandi dudit dispositif (100).

**14.** Utilisation selon la revendication 11, comprenant au moins les étapes qui consistent à :

> a) prévoir un dispositif (100) selon l'une des revendications 1 à 9,
> b) charger des cellules dans ledit dispositif (100),
> c) facultativement, incuber ledit dispositif (100),
> d) irradier ledit dispositif (100),
> e) facultativement, incuber ledit dispositif (100),
> f) déterminer l'influence de l'irradiation sur lesdites cellules en déterminant la superficie ou le volume d'agrégats individuels de cellules dans ledit dispositif (100) et/ou le nombre de cellules dans les agrégats individuels de cellules présents dans ledit dispositif (100), et comparer ladite superficie, ledit volume et/ou ledit nombre à une référence.

**15.** Trousse de production et de culture d'agrégats (111) de cellules, comprenant au moins un dispositif (100) selon au moins l'une des revendications 1 à 9 et une plaque multi-puits comprenant au moins deux puits, chaque puits comprenant l'un desdits dispositif (100).

**Figure 1**

**Figure 2**

**Figure 3**

**Figure 4**

Figure 5

**Figure 6**

**Figure 7**

| 360 cells | 900 cells | 2,400 cells | 6,000 cells | 16,000 cells | 40,000 cells |

## Figure 8

## Figure 9

**Figure 10**

**Figure 11**

**Figure 12**

**Figure 13**

**Figure 14**

**Figure 15**

**Figure 16**

**Figure 17**

Figure 18

Figure 19

**Figure 20**

**Figure 21**

**Figure 22**

Figure 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9106624 A1
- WO 2008106771 A1
- WO 2010142755 A2
- WO 0029110 A1
- EP 2230014 A1
- WO 9831466 A1
- WO 2009154466 A1
- US 2007235902 A1

### Non-patent literature cited in the description

- biology's new dimension. *NATURE,* August 2003, vol. 424, 870-872
- **FRIEDRICH J et al.** Spheroid-based drug screen: considerations and practical approach. *Nature Protocols,* 2009, vol. 4, 309-324
- **IVASCU A et al.** Rapid Generation of Single-Tumor Spheroids for High-Throughput Cell Function and Toxicity Analysis. *J Biomol Screen,* 2006, vol. 11, 922-932
- **TANG MD et al.** Molding of Three-Dimensional Microstructures of Gels. *J. AM. CHEM. SOC.,* 2003, vol. 125, 12988-12989
- **FUKUDA J et al.** Orderly Arrangement of Hepatocyte Spheroids on a Microfabricated Chip. *Tissue Engineering,* 2005, vol. 11, 1254-1262
- **NAPOLITANO AP et al.** Scaffold-free three-dimensional cell culture utilizing micromolded nonadhesive hydrogels. *BioTechniques,* 2007, vol. 43, 494-500